# EUROPEAN PATENT APPLICATION

(11) **EP 3 603 731 A1**
(43) Date of publication of application: **05.02.2020**
(21) Application number: 18778167.9
(22) Date of filing: 29.03.2018
(51) Int. Cl.: A61M 37/00

(54) **PERCUTANEOUS ADMINISTRATION DEVICE**

(30) Priority: 31.03.2017 JP 2017072234; 31.03.2017 JP 2017072235; 31.03.2017 JP 2017072236
(71) Applicant: Toppan Printing Co., Ltd., Tokyo 110-0016 (JP)
(72) Inventor: KODAMA, Yoshihiro, Tokyo 110-0016 (JP)
(74) Representative: TBK
(86) International application number: PCT/JP2018/013214
(87) International publication number: WO 2018/181700

(57) **Abstract**

A transdermal administration device includes an administration section and a support body. An administration section includes a base having a base surface, and a projection protruding from base surface and having a peripheral surface, the projection including a flow path which extends through the projection to the peripheral surface and is open to the peripheral surface. A support body includes a plurality of support sections configured to abut a target of administration. When viewed in a direction facing base surface, the plurality of support sections surround the base surface with a gap between the adjacent support sections and between the base surface and the support sections. The transdermal administration device is configured to displace the projection relative to the support sections from a position in a space surrounded by the plurality of support sections toward outside the space in an extending direction of the projection.

## Description

### [Technical Field]

The present invention relates to transdermal administration devices used for drug administration.

### [Background Art]

Using a microneedle device is a known method of administering a drug such as a vaccine into the body. A microneedle device includes a projection formed as a sharp point needle, which protrudes from a surface of a base. An administration method using a microneedle device is a drug administration technique by which the projection is punctured into the skin to create a hole in the skin so that a drug is intradermally delivered through the hole. Since the projection has a length that does not reach nerve cells in the dermis layer of the skin, the administration method using a microneedle device reduces pain caused by puncturing the skin compared with subcutaneous drug administration using an injection needle.

In one of the drug administration methods using a microneedle device, a device having a through hole that extends in an extending direction of the projection and penetrates the base and the projection is used, so that a liquid drug is intradermally delivered through the through hole (for example, see PTL 1). Such a microneedle device is, for example, attached to a syringe barrel for use as an injection needle. In drug administration, the drug loaded in an outer cylinder of the syringe barrel is pushed toward the projection by a plunger of the syringe barrel being pressed. Accordingly, the drug flows through the through hole, exits the projection at the tip, and is intradermally delivered.

### [Citation List]

### [Patent Literature]

PTL 1: JP 2015-70869 A

### [Summary of the Invention]

### [Technical Problem]

The skin may have wrinkles or sagging, or may have high elasticity. Accordingly, it may be difficult to puncture the projection into the skin to a sufficient depth corresponding to the length of the projection, or to hold the projection at a constant position inserted in the skin while the drug is administered. Therefore, it has been proposed to perform drug administration using a transdermal administration device that includes a microneedle device and a support body having an annular shape surrounding the microneedle device.

In drug administration using such a transdermal administration device, a user presses the skin with the support body so that a region of the skin surrounded by the support body is stretched. Then, the projection is punctured into that region, and the drug is pressurized toward the projection.

As the pressure is applied to the drug, the drug is released from the projection into the intradermal layer, and spreads around the projection. However, since the support body presses the skin, the drug is not likely to spread outside a region of the skin located under the support body. As a result, the amount of drug that can be injected into the intradermal layer is limited to a region surrounded by the support body.

The amount of drug injected can be increased by increasing the internal diameter of the annular support body. However, an increased internal diameter reduces the ability of the support body to stretch the skin in a center part of the ring, that is, at a position where the projection punctures the skin, and increases the size of the transdermal administration device.

The present invention is directed to provide a transdermal administration device that can increase the amount of drug injected into the skin.

### [Solution to Problem]

A transdermal administration device for addressing the above issues includes: an administration section including a base having a base surface, and a projection protruding from the base surface and having a peripheral surface, the projection including a flow path which extends through the projection to the peripheral surface and is open to the peripheral surface; and a support body including a plurality of support sections configured to abut a target of administration, wherein, when viewed in a direction facing the base surface, the plurality of support sections surround the base surface with a gap between the adjacent support sections and between the base surface and the support sections, and the transdermal administration device is configured to displace the projection relative to the support sections from a position in a space surrounded by the plurality of support sections toward outside the space in an extending direction of the projection.

With this configuration, during drug administration, the drug is restricted from spreading radially outward from the region surrounding the projection at regions located under the support sections, that is, regions pressed by the support sections. On the other hand, the drug is allowed to spread radially outward from the region surrounding the projection 22 at regions located under the gaps between the support sections. Therefore, compared with the case where the support body having a closed ring shape with no gap is used, the amount of drug injected into the skin can be increased.

The invention described below is directed to provide a transdermal administration device that enables smooth administration of a drug.

A transdermal administration device for addressing the above issues includes: a base having a support surface; and a projection protruding from the support surface and having a peripheral surface, wherein the projection includes one main flow path which extends through the projection from a proximal end to a distal tip end of the projection and a plurality of sub flow paths which extend from the main flow path toward the peripheral surface and are open to the peripheral surface, and an end of the main flow path adjacent to the proximal end of the projection in an extending direction of the main flow path is an open end which penetrates the support surface, and an end close to the distal tip end of the projection is closed.

With this configuration, since the projection includes the plurality of sub flow paths which are open to the peripheral surface of the projection, the drug supplied to the projection is distributed and released to a plurality of intradermal sites. The drug released from the projection into the intradermal layer spreads into the tissues in which cells are densely packed. Accordingly, the drug can more easily spread into the intradermal layer when distributed and released to a plurality of sites rather than when the drug is only released at a single site. Further, a resistance to the drug released from the projection into the intradermal layer can also be reduced. Therefore, smooth administration of the drug can be performed.

A transdermal administration device for addressing the above issues includes: a base having a support surface; and a projection protruding from the support surface and having a peripheral surface, wherein the projection includes a main flow path which extends through the projection from a proximal end to a distal tip end of the projection and a sub flow path which extends from the main flow path toward the peripheral surface and is open to the peripheral surface, an end of the main flow path adjacent to the proximal end of the projection in an extending direction of the main flow path is an open end which penetrates the support surface and an end close to the distal tip end of the projection is closed, and an area of a region defined by the sub flow path in a cross-section perpendicular to an extending direction of the sub flow path is 300 µm² or more.

With this configuration, an opening of the sub flow path is provided on the peripheral surface of the projection, and the drug is released from the opening into the intradermal layer. While the projection is inserted into the skin, the intradermal tissues spread in a width direction of the projection rather than a longitudinal direction. Accordingly, with the above configuration, the drug can easily spread into the intradermal layer without leaking into the hypodermis, compared with a conventional configuration in which an end of the through hole which extends in the longitudinal direction of the projection is open in the vicinity of the distal tip end of the projection. Further, when the area defined by the sub flow path described above is 300 µm² or more, the drug can be smoothly released from the sub flow path into the intradermal layer.

### [Advantageous Effects of the Invention]

According to the present invention, the amount of drug injected into the skin can be increased.

### [Brief Description of the Drawings]

Fig. 1 is a view illustrating a schematic cross-sectional structure of a transdermal administration device according to a first embodiment of the transdermal administration device.
Fig. 2 is a view illustrating a planar structure of an administration section and a support body in the transdermal administration device of the first embodiment.
Fig. 3 is a view illustrating a procedure of drug administration using the transdermal administration device of the first embodiment, in which the support body is placed on the skin.
Fig. 4 is a view illustrating a procedure of drug administration using the transdermal administration device of the first embodiment, in which the projection punctures the skin.
Fig. 5 is a view illustrating a procedure of drug administration using the transdermal administration device of the first embodiment, in which the drug is injected into the skin.
Fig. 6 is a view illustrating a procedure of drug administration using the transdermal administration device of the first embodiment, in which the drug is injected into the skin.
Fig. 7 is a view schematically illustrating the skin surface after the drug is administered using the transdermal administration device of the first embodiment.
Fig. 8 is a view illustrating a planar structure of the administration section and the support body of an additional example of the transdermal administration device of the first embodiment.
Fig. 9 is a view schematically illustrating the skin surface after the drug is administered using the additional example of the transdermal administration device.
Fig. 10 is a view illustrating a procedure of drug administration using the additional example of the transdermal administration device, in which the drug is injected into the skin.
Fig. 11 is a view illustrating a procedure of drug administration using the additional example of the transdermal administration device, in which the drug is injected into the skin.
Fig. 12 is a perspective view illustrating a perspective structure of the transdermal administration device of a modified example of the first embodiment.
Fig. 13 is a view illustrating an arrangement of support sections of the transdermal administration device of Example 1-1.
Fig. 14 is a view illustrating an arrangement of the support sections of the transdermal administration device of Example 1-2.
Fig. 15 is a view illustrating an arrangement of the support sections of the transdermal administration device of Example 1-3.
Fig. 16A is an image of a skin surface after administration is performed using the transdermal administration device of Example 1-1.
Fig. 16B is a schematic view illustrating an arrangement of a raised region and a recessed region in Fig. 16A.
Fig. 17A is an image of a skin surface after administration is performed using the transdermal administration device of Example 1-2.
Fig. 17B is a schematic view illustrating an arrangement of a raised region and a recessed region in Fig. 17A.
Fig. 18A is an image of a skin surface after administration is performed using the transdermal administration device of Example 1-3.
Fig. 18B is a schematic view illustrating an arrangement of a raised region and a recessed region in Fig. 18A.
Fig. 19A is a view illustrating a perspective structure of a transdermal administration device according to a second embodiment as viewed from the front.
Fig. 19B is a view illustrating a perspective structure of the transdermal administration device according to the second embodiment as viewed from the back.
Fig. 20A is a view illustrating a perspective structure of a main flow path and sub flow paths in the transdermal administration device of the second embodiment.
Fig. 20B is a view illustrating a cross-sectional structure of the transdermal administration device of the second embodiment, taken in a direction parallel to an extending direction of the sub flow paths.
Fig. 21 is a view illustrating a planar structure of the transdermal administration device of the second embodiment.
Fig. 22 is a view of a syringe barrel to which the transdermal administration device of the second embodiment is attached, which is illustrated with part of an outer cylinder of the syringe barrel removed.
Fig. 23A is a view illustrating a perspective structure of an additional example of the transdermal administration device of the second embodiment as viewed from the front.
Fig. 23B is a view illustrating a perspective structure of the additional example of the transdermal administration device of the second embodiment as viewed from the back.
Fig. 24 is a view illustrating a perspective structure of a main flow path and a sub flow path in the additional example of the transdermal administration device.
Fig. 25 is a view illustrating a planar structure of the additional example of the transdermal administration device.
Fig. 26A is a view illustrating a perspective structure of an additional example of the transdermal administration device of the second embodiment as viewed from the front.
Fig. 26B is a view illustrating a perspective structure of the additional example of the transdermal administration device of the second embodiment as viewed from the back.
Fig. 27 is a view illustrating a perspective structure of a main flow path and a sub flow path in the additional example of the transdermal administration device.
Fig. 28 is a view illustrating a planar structure of the additional example of the transdermal administration device.
Fig. 29 is a view illustrating another example of a planar structure of the additional example of the transdermal administration device.
Fig. 30 is a view illustrating a perspective structure of the transdermal administration device of a modified example of the second embodiment.
Fig. 31 shows an evaluation result of the strength of the projection for the transdermal administration devices of Examples 2-1 to 2-5 and Comparative Example 2-1.
Fig. 32A is a view illustrating a perspective structure of a transdermal administration device according to a third embodiment as viewed from the front.
Fig. 32B is a view illustrating a perspective structure of the transdermal administration device according to the third embodiment of the transdermal administration device as viewed from the back.
Fig. 33A is a view illustrating a perspective structure of a main flow path and a sub flow path in the transdermal administration device of the third embodiment.
Fig. 33B is a view illustrating a cross-sectional structure of the transdermal administration device of the third embodiment, taken in a direction parallel to an extending direction of a sub flow path.
Fig. 34 is a view illustrating another example of a cross-sectional structure of the transdermal administration device of the third embodiment.
Fig. 35 is a view illustrating a planar structure of the transdermal administration device of the third embodiment.
Fig. 36 is a view of a syringe barrel in which the transdermal administration device of the third embodiment is attached, which is illustrated with part of an outer cylinder of the syringe barrel removed.
Fig. 37 is a perspective view illustrating a perspective structure of the transdermal administration device of a modified example of the third embodiment.
Fig. 38 is a perspective view illustrating a perspective structure of the transdermal administration device of a modified example of the third embodiment.
Fig. 39 is a graph which shows the relationship between an area of the sub flow path and the time required to release pure water in Test Examples 1 to 11.
Fig. 40 is a graph showing a part of Fig. 39 in an enlarged manner.

### [Description of Embodiments]

### (First Embodiment)

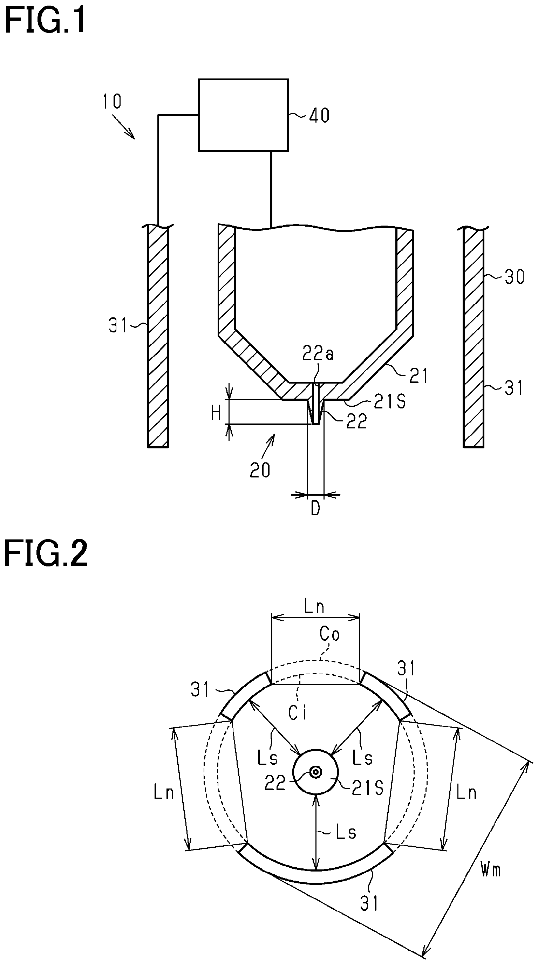

With reference to Figs. 1 to 11, a first embodiment of a transdermal administration device will be described.

### [Configuration of Transdermal Administration Device]

With reference to Figs. 1 and 2, a configuration of a transdermal administration device will be described.

As shown in Fig. 1, a transdermal administration device 10 includes an administration section 20, and a support body 30 for supporting the skin of an administration target, which is a target for drug administration. In the present embodiment, the administration section 20 is a microneedle device.

The administration section 20 includes a plate-shaped base 21 having a base surface 21S, and a projection 22 protruding from the base surface 21S.

The base surface 21S supports a proximal end of the projection 22. The shape of the base surface 21S is not specifically limited, and may be a circular or polygonal shape. For example, as shown in Fig. 1, the base 21 has a tubular shape extending from the base surface 21S in a direction opposite to the extending direction of the projection 22. In the tubular shape of the base 21, one end is an end face, which is the base surface 21S, and the other end is an opening. The base 21 may include, between both ends of the tubular shape, a portion having the outer diameter that varies gradually or in a stepwise manner. The inner side surface of the base 21 forms a flow path for supplying a liquid drug to the administration section 20. Alternatively, the base 21 may be a flat plate and connected to a tubular member that constitutes the above flow path.

The shape of the projection 22 is not specifically limited as long as it can puncture the skin. The projection 22 may have a conical or pyramid shape, or may have a cylindrical or prismatic shape. Alternatively, the projection 22 may have a shape formed by connecting the bottom of a cone or pyramid to the top of a cylinder or prismatic, or a shape formed by truncating a prismatic or cylindrical shape obliquely relative to the extending direction thereof, or a shape formed by connecting the bottom of a cone or pyramid to the top of a cylinder or prism, and truncating the structure obliquely relative to the extending direction thereof.

The projection 22 includes a through hole 22a that penetrates the projection 22 in the extending direction of the projection 22. One of the ends of the through hole 22a in the extending direction is open to the peripheral surface of the projection 22, and the other penetrates the base surface 21S. A space in the through hole 22a communicates with a space in the flow path, which is located on a side of the base surface 21S on which the projection 22 is not provided. The inner side surface of the through hole 22a forms a flow path for a drug, which is continuous from the above flow path. Further, the peripheral surface of the projection 22 is an outer peripheral surface of the projection, and includes the entire surface of the projection 22 located on the base surface 21S.

The administration section 20 may include a single projection 22 or a plurality of projections 22. When the administration section 20 includes a single projection 22, the projection 22 is preferably located at a center of the base surface 21S. Further, when the administration section 20 includes the plurality of projections 22, the plurality of projections 22 are arranged, for example, in a grid, circular, or coaxial pattern on the base surface 21S.

A length H of the projection 22 is a length from the base surface 21S to a distal tip end of the projection 22 in the extending direction of the projection 22, that is, a direction perpendicular to the base surface 21S. The length H of the projection 22 is preferably a length that penetrates the stratum corneum, which is the outermost layer of the skin, and does not reach the hypodermis, and specifically, preferably in the range of 200 µm or more and 2000 µm or less.

The projection 22 has a width D, which is a maximum length of the projection 22 in a direction parallel to the base surface 21S. For example, when the projection 22 has a conical shape, the width D of the projection 22 is a diameter of the circle of the bottom of the projection 22 on the base surface 21S. When the projection 22 has a quadrangular pyramid shape, the width D of the projection 22 is a length of the diagonal of the bottom of the projection 22. The width D of the projection 22 is preferably in the range of 150 µm or more and 1000 µm or less.

The support body 30 includes a plurality of support sections 31. Each support section 31 extends in the same direction as the extending direction of the projection 22, and the plurality of support sections 31 surround the administration section 20 with a gap between the base surface 21S and the support section 31.

An end of the support section 31 located on the same side as the distal tip end of the projection 22 in the extending direction of the projection 22 is a distal end of the support section 31. Positions of the distal ends of the plurality of support sections 31 are aligned with each other in the extending direction of the projection 22. In other words, the end faces on the distal ends of the respective support sections 31 are located in one plane which is parallel to the base surface 21S. A configuration of the end of the support section 31 opposite to the distal end is not specifically limited. For example, the ends opposite to the distal ends of the plurality of support sections 31 may be connected to one member.

The transdermal administration device 10 includes a drive unit 40, which is a mechanism to displace the administration section 20 relative to the support section 31 of the support body 30 in a direction from a proximal end toward the distal tip end of the projection 22. The drive unit 40 displaces the projection 22 from a position in the space surrounded by the plurality of support sections 31 toward outside the space in the extending direction of the projection 22 relative to the support sections 31. For example, the administration section 20 can be configured to reciprocate relative to the support body 30 in a direction perpendicular to the base surface 21S. The drive unit 40 can perform such displacement by using a mechanical structure such as a spring included in the drive unit 40, or by an electrical configuration driven by application of voltage.

As shown in Fig. 2, when viewed in a direction facing the base surface 21S, the plurality of support sections 31 surround the base surface 21S with a gap between the base surface 21S and the support sections 31. That is, the plurality of support sections 31 surround the projection 22 with a gap between the projection 22 and the support sections 31. Moreover, a gap is also provided between the adjacent support sections 31. In other words, when viewed in a direction facing the base surface 21S, the plurality of support sections 31 are positioned on a virtual ring surrounding the base surface 21S and the projection 22. In addition, when the administration section 20 includes a plurality of projections 22, the above ring surrounds a group of all the projections 22. Fig. 2 illustrates an example in which three support sections 31 are positioned on a ring surrounding the base surface 21S and the projection 22. Each support section 31 has a shape extending along the ring when viewed in the direction facing the base surface 21S.

A separation distance Ls for each of the support sections 31 is a minimum distance between the support section 31 and the base surface 21S as viewed in a direction facing the base surface 21S. The separation distance Ls may be different among each of the support sections 31, or may be the same among the plurality of support sections 31. The separation distance Ls is preferably in the range of 0.3 cm or more and 2.0 cm or less. Further, a minimum distance between the support section 31 and the projection 22 when viewed in the direction facing the base surface 21S is preferably in the range of 0.4 cm or more and 2.5 cm or less.

An adjacent distance Ln for each of adjacent support sections 31 is a minimum distance between the adjacent support sections 31 positioned along the ring surrounding the base surface 21S and the projection 22. The adjacent distance Ln may be different or the same between two adjacent support sections 31. That is, gaps between the adjacent support sections 31 may not be necessarily constant, or the support sections 31 may be arranged with equal intervals. The adjacent distance Ln is preferably in the range of 0.3 cm or more and 2.0 cm or less.

A maximum diameter Wm of the support body 30 is a maximum length of the support body 30 in a direction parallel to the base surface 21S. That is, the maximum diameter Wm is a distance between the ends of the support sections 31 which are farthest among the plurality of support sections 31. The maximum diameter Wm is preferably in the range of 0.8 cm or more and 5.0 cm or less. Further, the maximum diameter Wm is more preferably in the range of 1.0 cm or more and 2.5 cm or less.

When viewed in a direction facing the base surface 21S, a plurality of virtual circles that surround all the support sections 31, that is, a plurality of virtual circles in which all the support sections 31 are located therein, are referred to as first circles. Among the first circles, a circle having the smallest radius is an outermost circle Co. Further, when viewed in a direction facing the base surface 21S, a plurality of virtual circles about a center of the base surface 21S, in which any of the support sections 31 is not located inside the circle, are referred to as second circles. Among the second circles, a circle having the largest radius is an innermost circle Ci. The center of the base surface 21S is the center of gravity of the figure indicated by the outline of the base surface 21S. The outermost circle Co is in contact with at least one support section 31, and the innermost circle Ci is also in contact with at least one support section 31.

A region inside the outermost circle Co and outside the innermost circle Ci is a region between the outermost circle Co and the innermost circle Ci. A ratio of the area occupied by the distal ends of all the support sections 31 configured to abut the administration target to the area of the region between the outermost circle Co and the innermost circle Ci is an occupancy ratio Or. The occupancy ratio Or is preferably in the range of 0.2 or more and 0.8 or less. In other words, the occupancy ratio Or is a ratio of the sum of the distal end faces of the support sections 31 to the area of the outermost circle Co minus the area of the innermost circle Ci.

### [Method of Producing Transdermal Administration Device]

Materials and methods for producing a transdermal administration device 10 will be described.

Materials for forming the projection 22 are not specifically limited, and the projection 22 may be made of silicon or a metal material stainless steel, titanium, cobalt-chromium alloy, and magnesium alloy. Further, the projection 22 may be made of resin materials such as commodity plastics, medical grade plastics, and plastics for cosmetic products. Examples of the resin material include polyethylene, polypropylene, polystyrene, polyamide, polycarbonate, cyclic polyolefin, polylactic acid, polyglycolic acid, polycaprolactone, acrylic, urethane resin, aromatic polyether ketones, epoxy resin, and copolymer materials of these resins. Materials for the base 21 are not specifically limited, and the base 21 may be made of, for example, a material described above as the material for the projection 22.

The administration section 20 may be formed as a unitary molded product having the base 21 and the projection 22 integrally formed, or a combination of the base 21 and the projection 22 which are joined together after they are separately formed, or a combination of a metal material and a resin material. When the base 21 and the projection 22 are separately formed, or when the administration section 20 is formed of a combination of a metal material and a resin material, a sealing agent, adhesive, gasket, O-ring, or the like may also be used as necessary to tightly seal the separate components constituting the administration section 20.

The administration section 20 can be formed by, for example, machining the outer shape of the base 21 and the projection 22, and then forming the through hole 22a. Alternatively, when the administration section 20 is made of a resin material, the administration section 20 can be formed by injection molding by using a plurality of movable mold. Further, the administration section 20 can also be formed by a combination of injection molding and machining. For example, after the outer shapes of the base 21 and the projection 22 are formed by injection molding, the through hole 22a is formed by machining. Examples of the machining technique used for forming the through hole 22a include laser processing.

Materials for forming the support section 31 that constitutes the support body 30 are not specifically limited, and the support section 31 may be made of, for example, a metal material or a resin material. Examples of the metal material include aluminum, stainless steel, and brass. On the other hand, examples of the resin material include polyethylene, polypropylene, polystyrene, polyamide, polycarbonate, cyclic polyolefins, polylactic acid, polyglycolic acid, polycaprolactone, acrylic, urethane resin, aromatic polyether ketones, epoxy resin, and copolymer materials of these resins. The support section 31 may be formed by machining, injection molding, or the like according to the materials.

### [Effects]

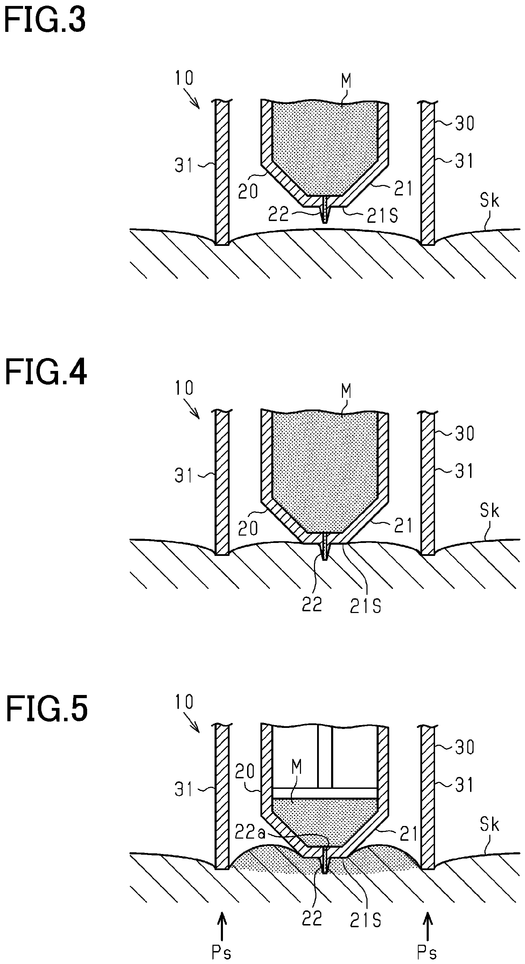

With reference to Figs. 3 to 7, usage and effects of the transdermal administration device 10 of the present embodiment will be described.

The tip of the projection 22 is set so as not to protrude from the distal end faces of the supports 31 of the support 30 immediately before the transdermal administration device 10 is used for drug administration. That is, the projection 22 is located within a space surrounded by the plurality of support sections 31. Further, the transdermal administration device 10 is filled with a liquid drug M.

As shown in Fig. 3, in drug administration, the support body 30 is first pressed against the skin Sk of the drug administration target. Accordingly, the distal end faces of the support sections 31 abut the skin Sk, and the support sections 31 press the skin Sk. As the support body 30 is pressed, the site of the skin Sk inside the region pressed by the support body 30 is stretched so that the stretched skin Sk is supported at a predetermined position.

As shown in Fig. 4, the administration section 20 is then directed downward. That is, the projection 22 is moved relative to the support body 30 in a direction directed from the proximal end to the distal tip end of the projection 22. Accordingly, the distal tip end of the projection 22 is punctured into the site of the skin Sk inside the region pressed by the support body 30. In addition, when punctured into the skin Sk, the projection 22 may be positioned within the space surrounded by the plurality of support sections 31, or may protrude from the space.

As shown in Fig. 5, pressure is then applied to the drug M loaded in the transdermal administration device 10. As a result, the drug M flows via the through hole 22a of the projection 22 and exits the projection 22 in the vicinity of the distal tip end, and is injected into the intradermal layer.

While the pressure is continuously applied to the drug M, the drug M is continuously released from the projection 22 and spreads into the intradermal layer. At the site of the skin Sk where the drug M has spread, the skin Sk swells and bulges due to the liquid drug M present in the intradermal layer. Since the tissues are compressed by the pressure applied from the support sections 31 at the site of the skin Sk pressed by the support sections 31, that is, pressed regions Ps located under the support sections 31, the drug M is not likely to spread. Further, at the pressed regions Ps, swelling of the skin Sk is restricted by the pressure applied from the support sections 31. Accordingly, in the region of the skin around the projection 22, where the pressed regions Ps are located at positions radially spaced from the center projection 22, the drug M mainly spreads radially inside the pressed regions Ps. In addition, swelling of the skin Sk is also restricted at a region pressed by the base surface 21S.

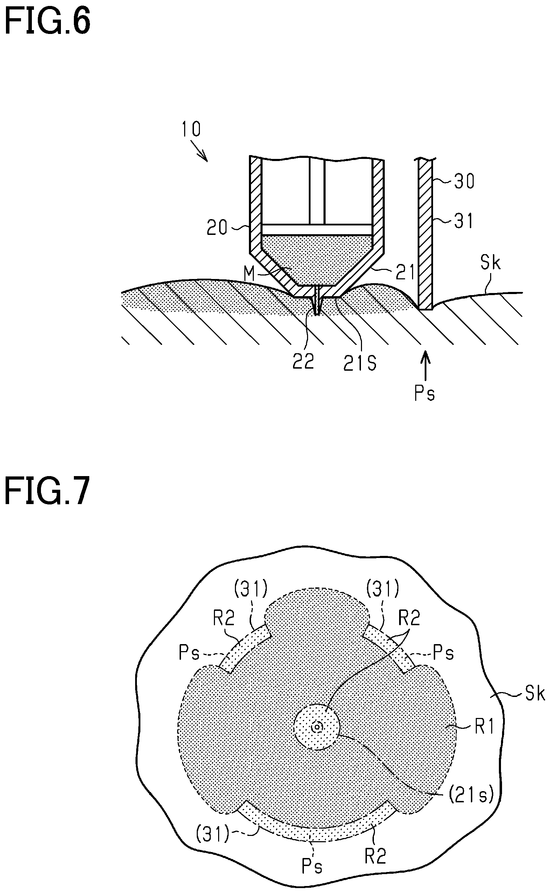

On the other hand, as shown in Fig. 6, in the region of the skin around the projection 22, where the pressed regions Ps are not located at positions radially spaced from the center projection 22, that is, under the gaps between the support sections 31, the drug M spreads radially outward according to the released amount of the drug M.

When the administration of the drug M is completed, the projection 22 is removed from the skin Sk, and the pressure applied to the skin Sk from the support body 30 is released.

Fig. 7 is a view schematically illustrating the surface of the skin, from which the transdermal administration device 10 is removed after the drug administration.

As shown in Fig. 7, when viewed in a direction facing the surface of the skin Sk, a raised region R1, which swells due to the injected drug, and recessed regions R2, which are recessed due to pressure marks left, are observed. Regions other than the raised region R1 and the recessed regions R2 remain flat, as before the drug administration.

In regions which have been pressed by the base surface 21S, and the regions which have been pressed by the distal end faces of the support sections 31, that is, the pressed regions Ps, swelling of the skin due to drug injection is restricted and the skin is recessed due to the pressure applied thereto. Therefore, the regions around each of these regions form the recessed region R2. That is, the recessed regions R2 are formed such that one recessed region R2 is positioned at the center, and the other recessed regions R2 are positioned on a ring about the center recessed region R2.

In the regions of the skin where the pressed regions Ps are located at positions radially spaced from the center projection 22, the raised region R1 is mainly formed radially inside the recessed regions R2, that is, inside the ring described above. On the other hand, in the regions of the skin where the regions under the gaps between the support sections 31 have been located at positions radially spaced from the center projection 22, the raised region R1 extends not only inside of the above ring but also to the outside of the above ring through the gaps between the recessed regions R2.

Thus, after the drug administration, a pattern made up of the raised region R1 and the recessed regions R2 is formed on the skin. This pattern gradually disappears as the drug is absorbed or spreads into the body.

According to the transdermal administration device 10 of the present embodiment, during drug administration, the drug is restricted from spreading radially outward from the region surrounding the projection 22 at regions under the support sections 31, while the drug is allowed to spread radially outward from the region surrounding the projection 22 at regions under the gaps between the support sections 31. Therefore, compared with the case where the support body having a closed ring shape with no gap is used, the amount of drug injected into the skin can be increased without expanding the ring of the support body.

Accordingly, when a large amount of drug, for example, 200 µL or more needs to be administered, drug administration can be performed without requiring a process such as concentration for reducing the amount of dose. Therefore, the transdermal administration device 10 of the present embodiment is particularly suited for administration of drugs of the amount of 200 µL or more, and administration of such drugs can be smoothly performed.

### [Other Examples of Support Body]

In the example described in the above description, three support sections 31 are provided. However, the number of support sections 31 of the support body 30 may be two, or four or more. Further, in the above description, the plurality of support sections 31 are positioned on a virtual ring surrounding the base surface 21S. However, the ring on which the plurality of support sections 31 are positioned may also be a rectangular shape or other polygonal shape, or an oval ring, or a shape formed by combination of straight lines and curved lines other than these figures. Further, the shape of the support section 31 as viewed in a direction facing the base surface 21S, that is, the shape of the distal end face of the support section 31, may not be necessarily a strip shape.

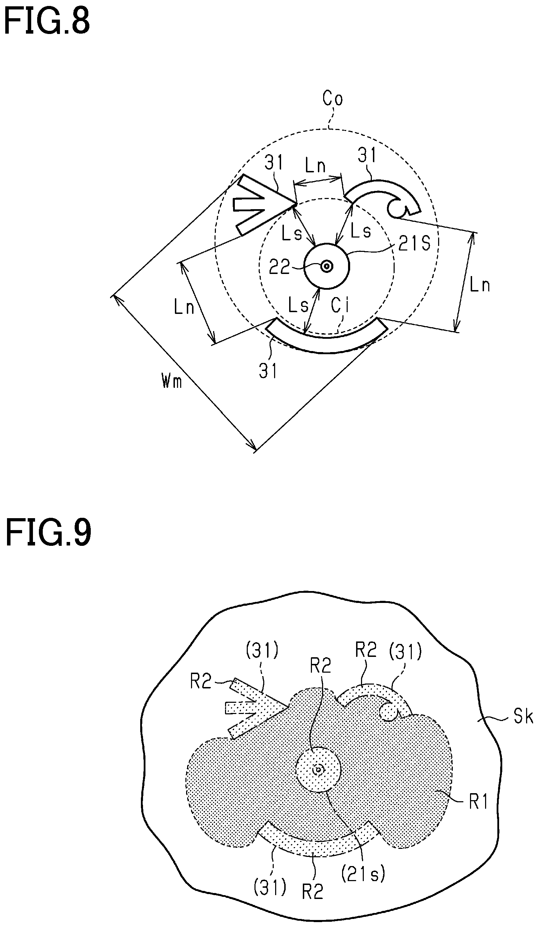

Fig. 8 illustrates an example in which three support sections 31 are shaped and arranged to form respective parts of a single pattern when viewed in a direction facing the base surface 21S. The three support sections 31 are configured to represent parts of the face, specifically, the eyebrows, eyes, and mouth. Furthermore, the base surface 21S is also configured to represent part of the face, specifically the nose. Thus, the support portions 31 and the base surface 21S form parts of the pattern. In this case as well, the separation distance Ls, the adjacent distance Ln, the maximum diameter Wm, and the occupancy ratio Or are defined in the same manner as those described in connection with Fig. 2.

Fig. 9 schematically illustrates the surface of the skin, from which the transdermal administration device 10 has been removed, after drug administration is performed by using the transdermal administration device 10 provided with the support body 30 and having the support sections 31 shaped and arranged as shown in Fig. 8.

As shown in Fig. 9, the recessed regions R2 are formed at regions pressed by the base surface 21S and the support sections 31. The raised region R1 is formed at a region surrounded by the support sections 31, and at regions extending outward from regions located under the gaps between the support sections 31. Thus, the raised region R1 and the recessed regions R2 provide a face-like pattern, in which the recessed regions R2 look like eye brows, eyes, nose, and mouth, and the raised region R1 look like cheeks and forehead.

As described above, when the plurality of support sections 31 and the base surface 21S are disposed at positions corresponding to the recessed areas among the raised and recessed areas of the pattern when viewed in a direction facing the base surface 21S, a mark is left on the skin of the administration target as a pattern composed of the raised and recessed areas after the drug administration. Accordingly, the discomfort of a recipient due to an injection mark being left and thus the discomfort of the recipient for drug administration can be reduced. Examples of the pattern include figures, pictures, designs, characters, symbols, numbers, and the like.

Further, the plurality of support sections 31 may be configured such that positions of some of the distal ends of the support sections 31, that is, some of the ends configured to abut the administration target, are not aligned in the extending direction of the projection 22. In other words, the distal end faces of all the support sections 31 may not be necessarily located in one plane parallel to the base surface 21S.

As the distal end face of the support section 31 is located at a lower position, that is, as the distal end face is located farther from the proximal end in the extending direction of the projection 22, a larger pressure is applied from the support section 31 to the skin, that is, swelling of the skin Sk is restricted by a larger force.

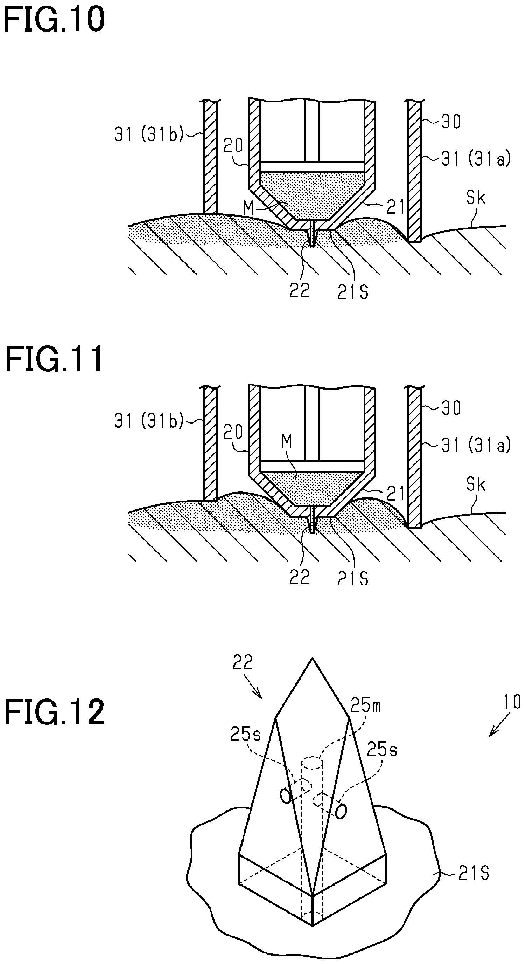

Fig. 10 illustrates an example in which the distal end faces of the support sections 31 of the support body 30 in the extending direction of the projection 22 are located at positions different from each other. For example, at the beginning of drug administration, the swelling of the skin Sk is still small since the injected amount of drug is small. Accordingly, at a region under the distal end face of a support section 31a which is located at the lowermost position, the drug is restricted from spreading radially outward from the region surrounding the projection 22 by the pressure applied from the support sections 31a. On the other hand, at a region under the distal end face of a support section 31b which is located at a relatively upper position, the drug is not likely to be restricted from spreading radially outward since the pressure applied by the support section 31b is weak.

As shown in Fig. 11, as the injected amount of drug increases and thus the swelling of the skin Sk increases, the drug is restricted from spreading radially outward by the pressure applied from the support section 31b at the region under the support section 31b. As a result, the degree of spread of the drug, the height of the asperities formed on the skin after the drug administration, and the like, can be varied among the regions located under the gaps between the support sections 31 in a radial direction, regions inside and outside the region under the support section 31a, and regions inside and outside the region under the support section 31b in the region surrounding the projection 22.

Thus, positions of the ends of the support sections 31 can be adjusted to adjust the timing and amount of pressure applied to the skin by the respective support sections 31. Accordingly, a mark left on the skin after drug administration can also be adjusted.

As described above, according to the first embodiment, the following effects can be achieved.
(1-1) The plurality of support sections 31 surround the base surface 21S and the projection 22 with a gap between the adjacent support sections 31 and between the base surface 21S and the support sections 31. Accordingly, during drug administration, the drug is restricted from spreading radially outward from the region surrounding the projection 22 at regions under the support sections 31, while the drug is allowed to spread radially outward from the region surrounding the projection 22 at regions under the gaps between the support sections 31. Therefore, compared with the case where the support body having a closed ring shape with no gap is used, the amount of drug injected into the skin can be increased.
(1-2) In the configuration in which the occupancy ratio Or is in the range of 0.2 or more and 0.8 or less, the support section 31 pressing the skin and the gap between the support sections 31 can have appropriate sizes. Accordingly, the skin can be successfully stretched by the support body 30, and an appropriate amount of drug can spread outward from under the gap between the support section 31.
(1-3) In the configuration in which the positions of the distal ends of the support sections 31 are aligned with each other in the extending direction of the projection 22, the skin tend to be uniformly stretched by the respective support sections 31. Accordingly, the skin can be successfully stretched by the support body 30, and the transdermal administration device 10 can be stably positioned relative to the skin.
(1-4) In the configuration in which the positions of some of the distal ends of the support sections 31 are not aligned in the extending direction of the projection 22, positions of the distal ends of the support sections 31 can be adjusted to adjust the timing and amount of pressure applied to the skin by the respective support sections 31. Accordingly, a mark left on the skin after drug administration can also be adjusted.
(1-5) In the configuration in which the plurality of support sections 31 are disposed to form respective parts of a single pattern when viewed in the direction facing the base surface 21S, the pattern composed of raised and recessed areas is left on the skin of the administration target after drug administration. Accordingly, the discomfort of a recipient due to an injection mark being left and thus the discomfort of the recipient for drug administration can be reduced.

### [Modified Examples]

The first embodiment can be implemented with modifications as described below.

The projection 22 is only required to have a flow path that is open to a peripheral surface of the projection 22, and may be different from the through hole 22a having the flow path penetrating the projection 22 in the extending direction thereof. For example, an administration section of a second embodiment and modified examples thereof, or an administration section of a third embodiment and modified examples thereof, may be used as the administration section. Specifically, for example, as shown in Fig. 12, the flow path may be a bent flow path including a main flow path 25m and a sub flow path 25s. The main flow path 25m is a flow path that extends in the extending direction of the projection 22, and one end of the main flow path 25m adjacent to the proximal end of the projection 22 penetrates the base surface 21S, and the other end close to the distal tip end of the projection 22 is closed. The sub flow path 25s extends from the main flow path 25m and is open to the peripheral surface of the projection 22. Furthermore, as shown in Fig. 12, the projection 22 may include a plurality of sub flow paths 25s. With this configuration, since the drug is released from the projection 22 in a direction in which the skin extends, the drug easily spreads into the intradermal layer.

In addition, the number of the sub flow paths 25s preferably matches the number of the gaps between the support sections 31, and each sub flow path 25s preferably extends in a direction toward a corresponding gap between the support sections 31 from the main flow path 25m when viewed in the direction facing the base surface 21S. With this configuration, the drug is released from the openings of the sub flow paths 25s toward the regions in the skin located under the gaps between the support sections 31, that is, the regions into which the drug easily spreads. Therefore, drug can efficiently spread into the intradermal layer.

Furthermore, Fig. 12 illustrates an example in which the projection 22 has a shape formed by connecting the bottom of a quadrangular pyramid to the top of a quadrangular prism, which is truncated obliquely relative to the extending direction thereof.
- The transdermal administration device 10 may include a drug and may be stored with the drug being filled, or may not include a drug and a drug may be supplied to the transdermal administration device 10 immediately before administration.
- The target to which the drug is administered by the administration section 20 is not limited to a human, but may also be another animal. Further, the configurations of the above embodiment and the modified examples can be appropriately combined for use.

### [Examples]

The transdermal administration device of the first embodiment will now be described by using specific examples.

### <Example 1-1>

A polycarbonate was used as a material for the administration section. A structure composed of a tubular base and a projection and having a through hole as a flow path for a drug was produced by injection molding. The projection had a shape formed by cutting a quadrangular pyramid, which was connected to the top of the quadrangular prism having a square bottom, in a direction oblique to the extending direction of the quadrangular pyramid. Thus, an administration section of Example 1-1 was obtained.

A polycarbonate was used as a material for the support section. Two support sections each having a shape corresponding to a part of a cylinder in the circumferential direction were formed by cutting. The two support sections were arranged on a ring surrounding the base surface of the administration section, and then the support body and the administration section were assembled together. Thus, a transdermal administration device of Example 1-1 was obtained.

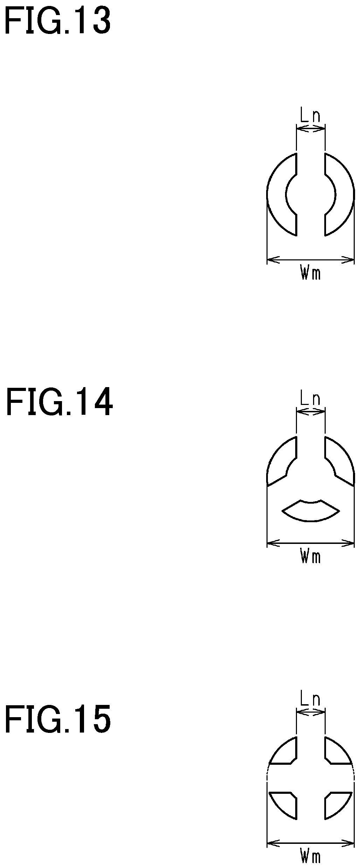

Fig. 13 illustrates an arrangement of the support sections in the transdermal administration device of Example 1-1. In the transdermal administration device of Example 1-1, the adjacent distance Ln was constant at 6 mm, and the maximum diameter Wm was 16 mm. Further, the base surface had a circular shape, the diameter was 3 mm, the separation distance Ls was constant at 4 mm, and the occupancy ratio Or was 0.75. Further, the length H of the projection was 750 µm.

### <Example 1-2>

The administration section having the same shape as in Example 1-1 was obtained by the same process as in Example 1-1. Further, three support sections each having a shape corresponding to a part of a cylinder in the circumferential direction were formed by the same process as in Example 1-1. The three support sections were arranged on a ring surrounding the base surface of the administration section, and then the support body and the administration section were assembled together. Thus, a transdermal administration device of Example 1-2 was obtained.

Fig. 14 illustrates an arrangement of the support sections in the transdermal administration device of Example 1-2. In the transdermal administration device of Example 1-2, the adjacent distance Ln was constant at 6 mm, and the maximum diameter Wm was 16 mm. Further, the base surface had a circular shape, the diameter was 3 mm, the separation distance Ls was constant at 4 mm, and the occupancy ratio Or was 0.63.

### <Example 1-3>

The administration section having the same shape as in Example 1-1 was obtained by the same process as in Example 1-1. Further, four support sections each having a shape corresponding to a part of a cylinder in the circumferential direction were formed by the same process as in Example 1-1. The four support sections were arranged on a ring surrounding the base surface of the administration section, and then the support body and the administration section were assembled together. Thus, a transdermal administration device of Example 1-3 was obtained.

Fig. 15 illustrates an arrangement of the support sections in the transdermal administration device of Example 1-3. In the transdermal administration device of Example 1-3, the adjacent distance Ln was constant at 6 mm, and the maximum diameter Wm was 16 mm. Further, the base surface had a circular shape, the diameter was 3 mm, the separation distance Ls was constant at 4 mm, and the occupancy ratio Or was 0.5.

### <Administration Test>

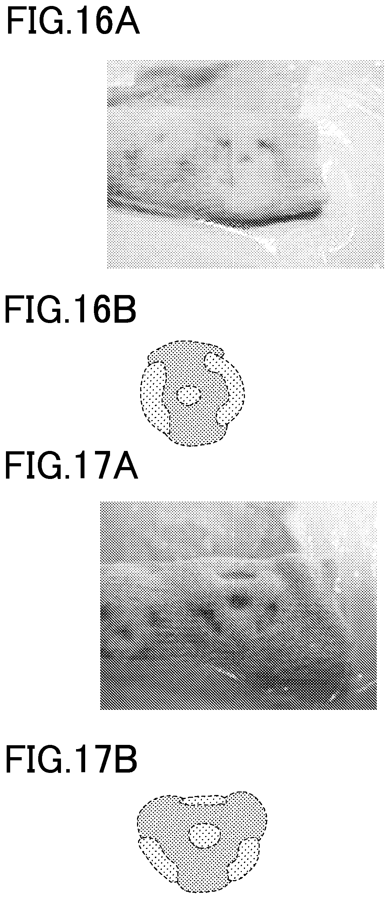

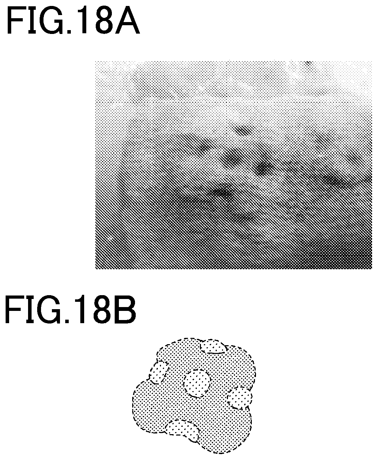

Drug administration tests were performed by using the transdermal administration devices of Example 1-1 to 1-3. Each transdermal administration device was filled with pure water as a drug. The support body was pressed against the skin taken from a 12-week-old Wistar rat, and the skin was punctured by the projection. Subsequently, the drug was pressurized toward the projection, and 600 µL drug was injected. After the administration of the drug, the transdermal administration device was removed from the skin, and the skin surface was observed. Fig. 16A is an image of the skin surface after the administration using the transdermal administration device of Example 1-1 was performed, and Fig. 16B is a schematic view illustrating the arrangement of the raised region and the recessed region in Fig. 16A. Fig. 17A is an image of the skin surface after the administration using the transdermal administration device of Example 1-2 was performed, and Fig. 17B is a schematic view illustrating the arrangement of the raised region and the recessed region in Fig. 17A. Fig. 18A is an image of the skin surface after the administration using the transdermal administration device of Example 1-3 was performed, and Fig. 18B is a schematic view illustrating the arrangement of the raised region and the recessed region in Fig. 18A.

As shown in Figs. 16 to 18, in each of the examples, the raised region extends outward beyond the region under the gap between the support sections. Therefore, compared with the case where the support body having a closed ring shape is used, it is suggested that the amount of drug injected into the skin can be increased.

### (Second Embodiment)

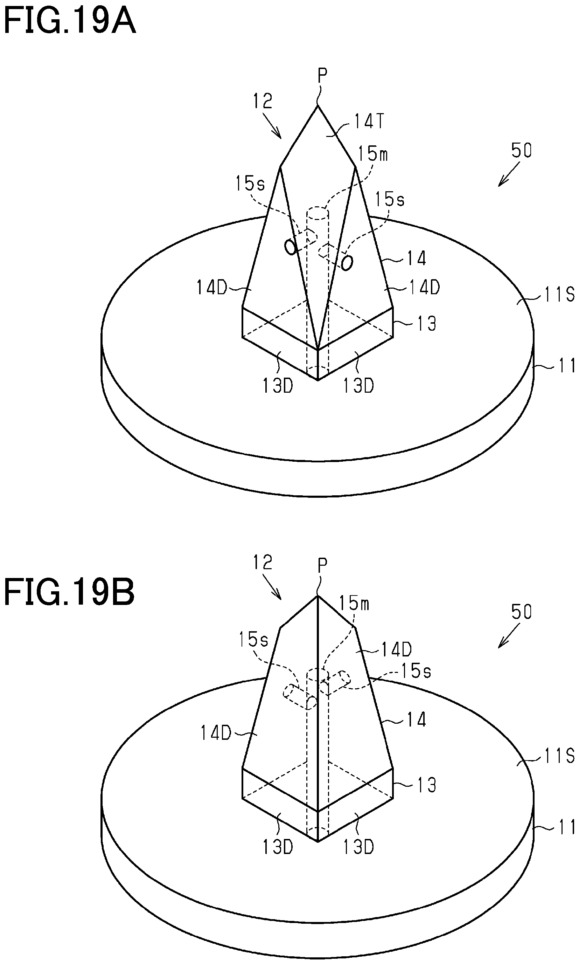

With reference to Figs. 19 to 29, a second embodiment of the transdermal administration device will be described.

### [Problem to be Solved by Second Embodiment]

In the administration method using a conventional microneedle device, the distal tip end of the projection is located in the intradermal layer of the skin during drug administration, in contrast to subcutaneous injection by which the distal tip end of the needle reaches the hypodermis. The intradermal layer has higher tissue density than the hypodermis, and the through hole of the projection has a relatively small diameter compared with that of typical injection needles, and further, the skin tissues may occasionally enter the through hole. For these reasons, a smooth flow of the drug from the through hole into the intradermal layer may be disturbed. If smooth administration of the drug into the intradermal layer is disturbed, the force required to press the drug and the time required to administer the drug increase. Accordingly, the burden on the person administering the drug and the recipient of the drug increase, and the amount of the drug intradermally administered decreases due to leakage of the drug to the surface of the skin or to the hypodermis.

The second embodiment is directed to provide a transdermal administration device that enables smooth administration of a drug.

### [Overall Configuration of Transdermal Administration Device]

The transdermal administration device of the second embodiment may include at least an administration section 50. In the second embodiment, the administration section 50 is a microneedle device.

As shown in Figs. 19A and 19B, the administration section 50 includes a base 11 having a support surface 11S, and a projection 12 protruding from the support surface 11S. The support surface 11S supports a proximal end of the projection 12. The shape of the support surface 11S is not specifically limited, and may be a circular or polygonal shape. The base 11 may be a flat plate shape as shown in Figs. 19A and 19B, or may be a tubular shape extending from the support surface 11S in a direction opposite to the extending direction of the projection 12. The base 11 may include a structure such as groove or boss on the outer periphery of the base 11 or the like for connecting the administration section 50 to a device for supplying a liquid drug to the administration section 50.

The projection 12 includes a columnar portion 13 having a cylindrical or prismatic shape extending from the support surface 11S, and a pyramidal portion 14 having a conical or pyramid shape extending from the top of the columnar portion 13 and truncated obliquely relative to the extending direction of the conical or pyramid shape.

Specifically, the columnar portion 13 has a quadrangular prism shape, and has four side faces 13D extending from a square bottom defined on the support surface 11S. The side face 13D is perpendicular to the support surface 11S. Further, the pyramidal portion 14 has a shape formed by truncating a quadrangular pyramid obliquely relative to the extending direction thereof. Accordingly, the pyramidal portion 14 has four side faces 14D and one top facet 14T, whose edges are shared with each of the side faces 14D.

The side faces 14D are inclined relative to the support surface 11S, and each side face 14D shares one edge with one side face 13D. The top facet 14T is also inclined relative to the support surface 11S, and all the edges included in the top facet 14T are also inclined relative to the support surface 11S. As shown in the example in Figs. 19A and 19B, among the vertices included in the top facet 14T, the vertex located closest to the base 11, that is, the vertex located closest to the proximal end of the projection 12, is located at the end in contact with the proximal end of the pyramidal portion 14, that is, the end in contact with the columnar portion 13. In this configuration, each side face 14D which shares the vertex of the top facet 14T located closest to the base 11 with the top facet 14T has a triangular shape.

The top facet 14T has a shape symmetrical to a straight line connecting the vertex closest to the base 11 and the vertex farthest from the base 11, and the vertex farthest from the base 11 constitutes an apex P, which is the apex of the projection 12.

The peripheral surface of the projection 12 is composed of the above four side faces 13D, four side faces 14D, and the top facet 14T. A length of the projection 12 from the support surface 11S is largest at the apex P.

The projection 12 includes one main flow path 15m which extends therein from the proximal end toward the distal tip end of the projection 12, and a plurality of sub flow paths which extend from the main flow path 15m toward the peripheral surface of the projection 12. The main flow path 15m communicates with each of the sub flow paths 15s, and both the main flow path 15m and the sub flow paths 15s define a space therein for allowing a fluid to flow in the projection 12. The main flow path 15m penetrates the support surface 11S and communicates with the outside of the administration section 50, while the sub flow path 15s is open to the peripheral surface of the projection 12. Figs. 19A and 19B show an example in which the projection 12 has two sub flow paths 15s.

The administration section 50 may include a single projection 12 or a plurality of projections 12. When the administration section 50 includes a single projection 12, the projection 12 is preferably located at a center of the support surface 11S. Further, when the administration section 50 includes a plurality of projections 12, the plurality of projections 12 are arranged, for example, in a grid, circular, or coaxial pattern on the support surface 11S.

### [Detailed Configuration of Flow Path]

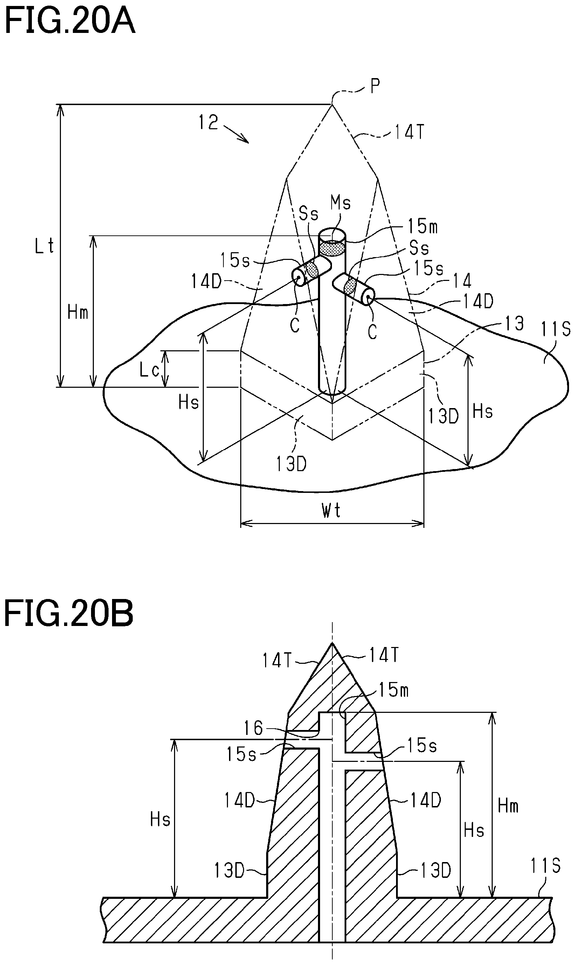

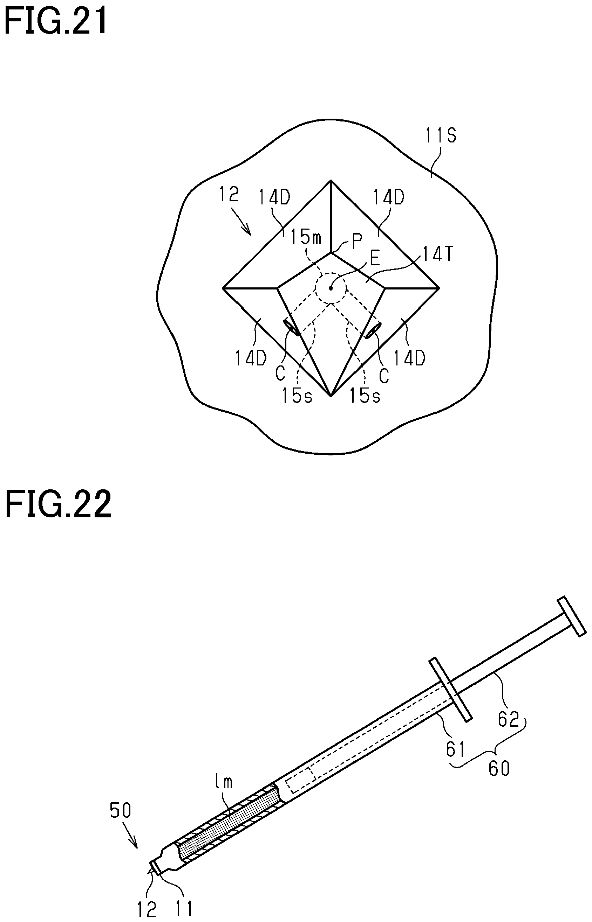

With reference to Figs. 20 and 21, a detailed configuration of the main flow path 15m and the sub flow path 15s will be described. Fig. 20A is a view in which an outline of the main flow path 15m and the sub flow path 15s are indicated by the solid line, and an outline of the projection 12 is indicated by the double dotted and dashed line. Fig. 20B is a view illustrating a cross-section of the projection 12 taken along the extending direction of the sub flow paths 15s in which a straight line passing through the center of the main flow path 15m is taken as a boundary.

As shown in Figs. 20A and 20B, the main flow path 15m is located in a center part of the projection 12, and extends in a longitudinal direction of the projection 12, that is, in a direction perpendicular to the support surface 11S. In the extending direction of the main flow path 15m, one of the ends of the main flow path 15m adjacent to the proximal end of the projection 12 is an open end. For example, the main flow path 15m penetrates the base 11 and is open to a surface of the base 11 on a side opposite to the support surface 11S. On the other hand, in the extending direction of the main flow path 15m, the other of the ends of the main flow path 15m close to the distal tip end of the projection 12 is closed. That is, the end of the main flow path 15m close to the distal tip end of the projection 12 does not reach the top facet 14T of the projection 12.

The sub flow paths 15s extend in a width direction of the projection 12, that is, in a direction parallel to the support surface 11S. One of the ends of the sub flow path 15s in the extending direction is connected to the main flow path 15m, and the other of the ends is open to the side face 14D of pyramidal portion 14 of the projection 12. Each of the plurality of sub flow paths 15s is open to a different one of the four side faces 14D. In other words, the number of sub flow paths 15s open to each side face 14D is 1 or less. The side face 13D of the top facet 14T and the columnar portion 13 do not include an opening which is an end of the flow path.

The main flow path 15m and the sub flow paths 15s constitute flow paths in the projection 12 for a fluid flowing via the opening end of the main flow path 15m toward the openings of the sub flow paths 15s, and the flow paths are bent at connecting sections between the main flow path 15m and the sub flow path 15s.

A region defined by each of the main flow path 15m and the sub flow path 15s in a cross-section perpendicular to the extending direction of the flow path is a circular shape. In a cross-section perpendicular to the extending direction of the main flow path 15m, an area of the region defined by the main flow path 15m is a main flow path area Ms, and the main flow path area Ms is constant in each main flow path 15m. In each of the sub flow paths 15s, an area of the region defined by the sub flow path 15s in a cross-section perpendicular to the extending direction of the sub flow path 15s is a sub flow path area Ss, and the sub flow path area Ss is constant in each sub flow path 15s. Further, the sub flow path areas Ss of the respective sub flow paths 15s are preferably the same.

When a resistance that a fluid flowing in the main flow path 15m receives per unit length is a main flow path resistance Mr, and a resistance that a fluid flowing in the sub flow path 15s per unit length is a sub flow path resistance Sr, the sum of the sub flow path resistances Sr for all the sub flow paths 15s is preferably equal to the main flow path resistance Mr. Specifically, the sum of the sub flow path areas Ss of all the sub flow paths 15s is preferably equal or so close to the main flow path area Ms that the sum of the sub flow path resistances Sr and the main flow path resistance Mr can be regarded as equal.

In order to smoothly release the drug from the sub flow path 15s into the intradermal layer, the sub flow path area Ss is preferably 300 µm² or more, and more preferably 700 µm² or more. Further, the sub flow path area Ss is preferably 8000 µm² or less, and more preferably 2000 µm² or less. When the sub flow path area Ss is 8000 µm² or less, appropriate administration of the drug into the intradermal layer of a human, and even of a small animal as an administration target of a test, is possible since the size of the opening of the sub flow path 15s relative to the thickness of the skin is not too large.

A length Lt of the projection 12 is a length in the longitudinal direction of the projection 12, that is, from the support surface 11S to the apex P of the projection 12 in a direction perpendicular to the support surface 11S. The length Lt of the projection 12 is preferably a length that penetrates the stratum corneum, which is the outermost layer of the skin, and does not reach the hypodermis, and specifically, preferably in the range of 200 µm or more and 2000 µm or less.

Further, a length Lc of the columnar portion 13 is a length from the support surface 11S of the projection 12 to the top of the columnar portion 13 in the longitudinal direction of the projection 12, and the length Lc of the columnar portion 13 is preferably 1/20 or more and 1/2 or less of the length Lt of the projection 12.

A width Wt of the projection 12 is a maximum length of the projection 12 in the width direction of the projection 12, that is, in a direction parallel to the support surface 11S. That is, the width Wt of the projection 12 is a length of a diagonal of a square formed by the bottom of the projection 12, defined on the support surface 11S. A width Wt of the projection 12 is preferably in the range of 150 µm or more and 1000 µm or less.

A height Hm of the main flow path 15m is a length of the main flow path 15m from the support surface 11S in the longitudinal direction of the projection 12, that is, a length of the main flow path 15m from the support surface 11S to the distal tip end of the projection 12. The height Hm of the main flow path 15m is smaller than the length Lt of the projection 12.

A height Hs of the sub flow path 15s is a length from the support surface 11S to a center C of the opening of the sub flow path 15s in the longitudinal direction of the projection 12. The center C of the opening is the center of gravity of the figure formed by a region defined by the opening, and is located on a straight line passing through the center of gravity of the region defined by the sub flow path 15s in the cross-section perpendicular to the extending direction of the sub flow path 15s. For example, in the case where the sub flow path 15s is circular in each cross-section and the sub flow path 15s is a cylinder, the center C of the opening of the sub flow path 15s is located on the straight line passing through the center of the circle, that is, on the center axis of the cylinder.

The heights Hs of the plurality of sub flow paths 15s are different from each other. Among the heights Hs of the plurality of sub flow paths 15s, the smallest height Hs, that is, the height Hs of the sub flow path 15s located closest to the support surface 11S, is preferably 100 µm or more. In other words, the center C of the sub flow path 15s closest to the support surface 11S is preferably separated from the support surface 11S by 100 µm or more. When the smallest height Hs is 100 µm or more, the drug supplied to the projection 12 is prevented from flowing out of the projection 12a at a position near the support surface 11S, that is, a position near the surface of the skin. Accordingly, the drug intended to be administered into the intradermal layer is prevented from leaking onto the skin. Further, among the heights Hs of the plurality of sub flow paths 15s, the smallest height Hs, that is, the height Hs of the sub flow path 15s located closest to the support surface 11S, is preferably 700 µm or less.

In addition, it is preferred that the region in each sub flow path 15s does not include a portion corresponding to the region in the other sub flow paths 15s in the longitudinal direction of the projection 12, in other words, the entire region of each sub flow path 15s is located at a position different from that of the entire region of the other sub flow paths 15s in the longitudinal direction of the projection 12.

Among the heights Hs of the plurality of sub flow paths 15s, the largest height Hs, that is, the height Hs of the sub flow path 15s located farthest from the support surface 11S, is preferably smaller than the height Hm of the main flow path 15m. In the sub flow path 15s farthest from the support surface 11S, a distance from an end connected to the main flow path 15m to the proximal end of the projection 12 is preferably smaller than a distance from the distal end of the main flow path 15m to the proximal end of the projection 12. In other words, in the longitudinal direction of the projection 12, the main flow path 15m extends toward the distal tip end of the projection 12 beyond the sub flow path 15s located farthest from the support surface 11S, and a stepped portion 16 is preferably formed at the connecting section between the sub flow path 15s and the main flow path 15m.

Accordingly, compared with a configuration in which the sub flow path 15s extends from a position of the same height as the distal end of the main flow path 15m, high precision is not required for positioning of the main flow path 15m and the sub flow path 15s when the administration section 50 is produced by forming the main flow path 15m and then forming the sub flow path 15s.

As shown in Fig. 21, when viewed in the direction facing the support surface 11S, the apex P of the projection 12 is located on an edge of the top facet 14T. When viewed in the direction facing the support surface 11S, the plurality of sub flow paths 15s extend from the main flow path 15m in directions different from each other. That is, the plurality of sub flow paths 15s do not overlap each other in the longitudinal direction of the projection 12. Further, the extending directions of the sub flow paths 15s are directions extending from a center E of the main flow path 15m to the respective centers C of the openings of the sub flow paths 15s. In addition, when viewed from the direction facing the support surface 11S, the openings of the plurality of sub flow paths 15s are located at positions different from each other, in other words, the centers C of the openings of the plurality of sub flow paths 15s are located at positions different from each other.

As shown in Figs. 19 to 21, two sub flow paths 15s of the projection 12 are open to different ones of the four side faces 14D, and two side faces 14D to which the sub flow paths 15s are open are adjacent to each other. The side faces 14D to which the sub flow paths 15s are open are the side faces 14D which do not include the apex P of the projection 12 and share an edge of the top facet 14T located on the lower side of the slope, that is, the edge close to the proximal end of the projection 12, with the top facet 14T. In other words, when four side faces 14D are divided into two side faces 14D closer to the apex P and two side faces 14D farther from the apex P, the sub flow paths 15s are open to the side faces 14D farther from the apex P. The side face 14D closer to the apex P is the side face 14D that shares an edge of the top facet 14T which includes the apex P with the top facet 14T, and the side face 14D farther from the apex P is the side face 14D that shares an edge of the top facet 14T which does not include the apex P with the top facet 14T.

### [Effects]

With reference to Fig. 22, effects of the administration section 50 of the present embodiment will be described.

As shown in Fig. 22, the administration section 50 is mounted on an end of an outer cylinder 61 of a syringe barrel 60. In administration of a liquid drug, the administration section 50 is pressed against the skin of an administration target of the liquid drug to puncture the skin using the projection 12. Then, while the projection 12 is punctured into the skin, the plunger 62 is pushed into the administration section 50. As the plunger 62 is pushed, a liquid drug lm loaded in the outer cylinder 61 is supplied into the main flow path 15m of the projection 12, and the drug lm is further fed from the main flow path 15m into the sub flow path 15s. Then, the drug lm flows out from the opening on the side face 14D of the projection 12 and flows into the skin.

Here, in the case where the projection has one through hole which penetrates the projection and is opened at the distal tip end of the projection as in the conventional art, or in the case where only one sub flow path 15s is provided, and thus only one outlet for the drug is provided, the drug is only released at a single site in the skin from the projection. On the other hand, according to the present embodiment, the projection 12 includes a plurality of sub flow paths 15s, and thus a plurality of outlets for the drug. Accordingly, the drug is distributed and released to a plurality of sites in the skin from the projection 12. Since the flow of the released drug receives a large resistance from the tissues in which cells are densely packed, the amount of the drug spreading from one position into the tissues per unit time is limited by this resistance. In this respect, with the configuration in which the drug is distributed and released from a plurality of positions, it is possible to increase the amount of drug spreading in the skin per unit time.

Further, the projection 12 is inserted into the skin from the apex P in the longitudinal direction. Therefore, compared with the conventional configuration in which the end of the through hole is open in the vicinity of the distal tip end of the projection, the skin tissue is less likely to enter the drug flow path in the puncture process in the configuration of the present embodiment in which the ends of the sub flow paths 15s which extend in directions different from the longitudinal direction of the projection 12 are open to the peripheral surface of the projection 12.

Further, while the drug is released in the longitudinal direction of the projection in the conventional configuration in which the end of the through hole is open in the vicinity of the distal tip end of the projection, the drug is released in the width direction of the projection 12 from the projection 12 of the present embodiment. While the projection 12 is inserted into the skin, the intradermal tissues spread in the width direction of the projection 12 rather than the longitudinal direction. Accordingly, with the above configuration, the drug can easily spread into the intradermal layer without leaking into the hypodermis compared with the conventional configuration.

Thus, according to the administration section 50 of the present embodiment, smooth administration of the drug can be performed. As a result, it is possible to prevent an increase in the burden on the person administering the drug and the recipient of the drug since the force required to press the drug and the time required to administer the drug are prevented from increasing. Further, since leakage of the drug to the surface of the skin or to the hypodermis due to the limitation on the amount of the drug that can spread into the intradermal layer is reduced, it is possible to prevent a decrease in the amount of the drug intradermally administered due to the drug leaking to a site different from the intended position.

In addition, the projection 12 is easily punctured into the skin since the projection 12 has the pyramidal portion 14 sharpened toward the distal tip end. Further, since the projection 12 has the columnar portion 13, the diameter of the hole created by the projection is prevented from expanding near the surface of the skin, compared with the configuration in which the projection is composed of only the pyramidal portion 14, that is, in which the side face inclined relative to the support surface 11S extends to the support surface 11S. As a result, the drug intradermally administered is less likely to leak onto the surface of the skin. In particular, since the sub flow path 15s is open to the side face 14D of the pyramidal portion 14, and the side face 13D of the columnar portion 13 does not include the opening, which is the end of the sub flow path 15s, the effect of preventing drug leakage onto the surface of the skin can be improved.

In addition, since the heights Hs of the plurality of sub flow paths 15s are different from each other, the plurality of sub flow paths 15s are prevented from being arranged in the same plane extending in the width direction of the projection 12. As a result, compared with a configuration in which the plurality of sub flow paths 15s are arranged in the width direction of the projection 12, that is, a configuration in which the channels formed in the projection 12 are arranged in the width direction of the projection 12, it is possible to prevent the strength of the projection 12 from being significantly reduced at a specific position in the longitudinal direction of the projection 12. In addition, since the plurality of sub flow paths 15s extend in directions different from each other when viewed in the direction facing the support surface 11S, the plurality of sub flow paths 15s are prevented from being arranged in the same plane extending in the longitudinal direction of the projection 12. As a result, compared with a configuration in which the plurality of sub flow paths 15s are arranged in the longitudinal direction of the projection 12, that is, a configuration in which the channels formed in the projection 12 are arranged in the longitudinal direction of the projection 12, it is possible to prevent the strength of the projection 12 from being significantly reduced at a specific position in the width direction of the projection 12. Therefore, since the projection 12 becomes less likely to be deformed during puncture into skin, the projection 12 can be easily punctured into the skin to the depth according to the length of the projection 12. Therefore, it is possible to precisely administer the drug to a desired position in the intradermal layer.

Further, in a configuration in which the apex P is located on the edge of the top facet 14T, when the projection 12 is punctured into the skin, the projection 12 undergoes a force pressing against the top facet 14T, that is, a force that causes the projection 12 to be inclined toward a side where the apex P is located, rather than toward the center of the projection 12. Therefore, in the configuration in which two sub flow paths 15s are provided and the sub flow paths 15s are open only to the side faces 14D located farther from the apex P, compared with a configuration in which the sub flow paths 15s are open to the side faces 14D located closer to the apex P, the projection 12 has an increased strength against a force that causes the projection 12 to be inclined toward a side where the apex P is located. Accordingly, deformation of the projection 12 can be reduced.

### [Other Examples of Transdermal Administration Device]

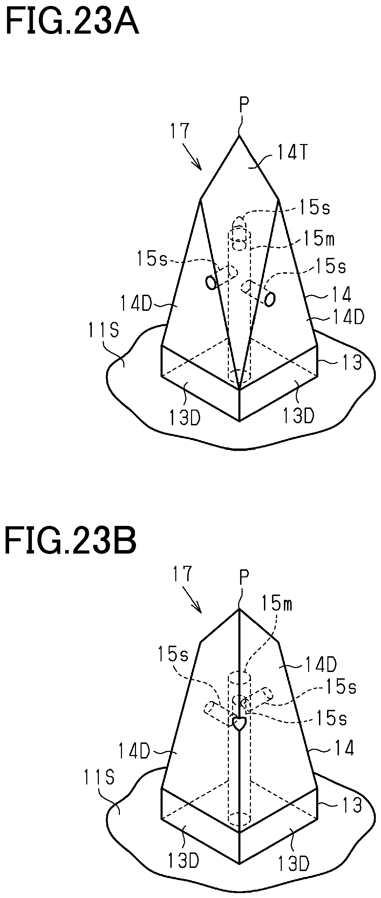

In the above description, a projection 12 having the two sub flow paths 15s is described. However, the number of the sub flow paths 15s is not limited as long as it is two or more. With reference to Figs. 23 to 29, an example having three or more sub flow paths 15s and an example having four or more sub flow paths 15s will be described.

A projection 17 shown in Figs. 23A and 23B includes three sub flow paths 15s. Each of the three sub flow paths 15s is open to a different one of the four side faces 14D.

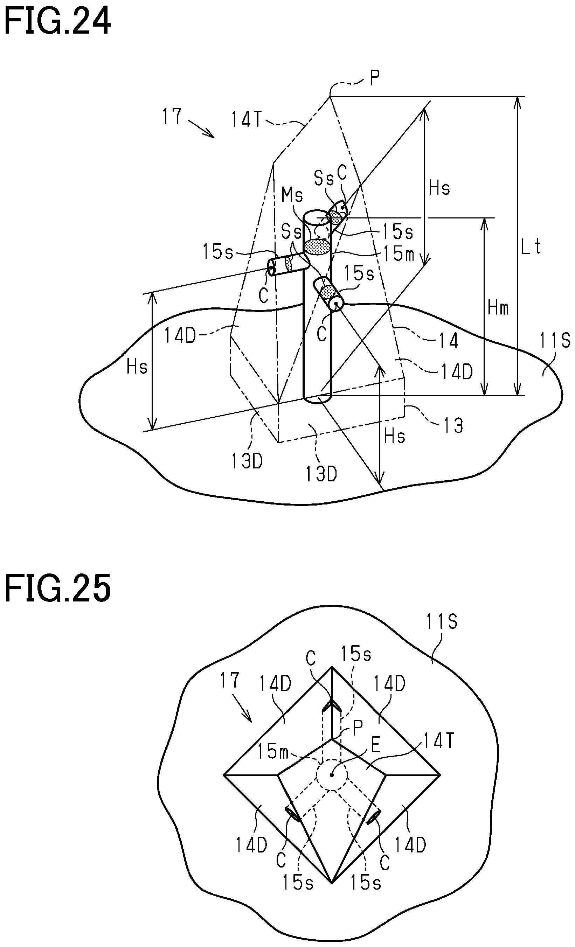

As shown in Fig. 24, the heights Hs of the three sub flow paths 15s are different from each other. In the longitudinal direction of the projection 17, the centers C of the openings of three sub flow paths 15s are preferably arranged with equal intervals therebetween. That is, among the heights Hs of three sub flow paths 15s, the median height Hs is preferably an average of the largest height Hs and the smallest height Hs. With this configuration, the drug can be more evenly distributed and released in the longitudinal direction of the projection 17. The sum of the sub flow path resistances Sr of three sub flow paths 15s is preferably equal to the main flow path resistance Mr. The sum of the sub flow path areas Ss of three sub flow paths 15s is preferably equal or so close to the main flow path area Ms that the sum of the sub flow path resistances Sr and the main flow path resistance Mr can be regarded as equal.

As shown in Fig. 25, when viewed in the direction facing the support surface 11S, three sub flow paths 15s extend from the main flow path 15m in directions different from each other. Two of the three sub flow paths 15s are each open to respective ones of the four side faces 14D which do not include the apex P of the projection 12. The remaining one of the three sub flow paths 15s is open at a position across two side faces 14D which share the apex P, that is, on the boundary between two side faces 14D located closer to the apex P among the four side face 14D. With this configuration as well, the number of sub flow paths 15s open to each one of the four side faces 14D is 1 or less.

Further, the three sub flow paths 15s maybe open to each of the two side faces 14D farther from the apex P and one of the two side faces 14D closer to the apex P. However, as shown in Figs. 23 to 25, the drug can be more evenly distributed and released in the width direction of the projection 17 in the configuration in which the three sub flow paths 15s are open to two side faces 14D located farther from the apex P and at a position across the two side faces 14D located closer to the apex P.

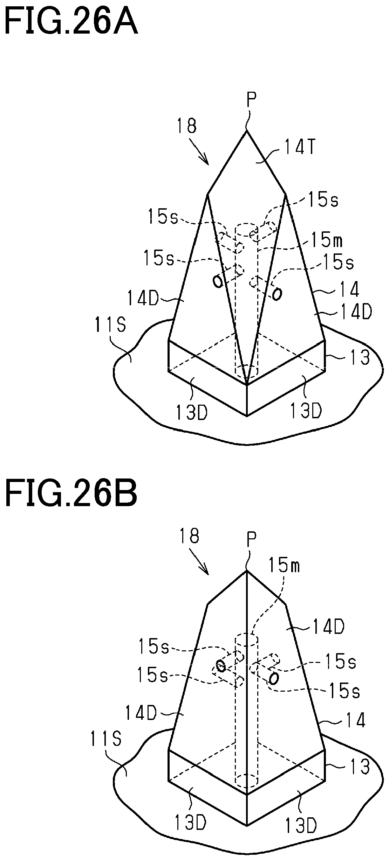

A projection 18 shown in Figs. 26A and 26B includes four sub flow paths 15s. Each of the four sub flow paths 15s is open to a different one of the four side faces 14D.

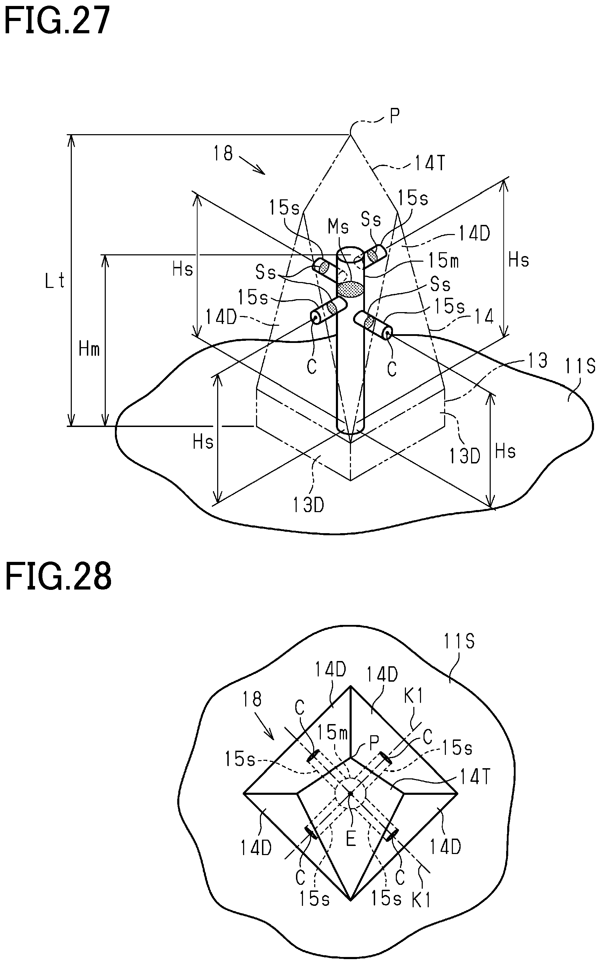

As shown in Fig. 27, the heights Hs of the four sub flow paths 15s are different from each other. In the longitudinal direction of the projection 18, the centers C of the openings of four sub flow paths 15s are preferably arranged at equal intervals. With this configuration, the drug can be more evenly distributed and released in the longitudinal direction of the projection 18. The sum of the sub flow path resistances Sr of four sub flow paths 15s is preferably equal to the main flow path resistance Mr. The sum of the sub flow path areas Ss of four sub flow paths 15s is preferably equal or so close to the main flow path area Ms that the sum of the sub flow path resistances Sr and the main flow path resistance Mr can be regarded as equal.

As shown in Fig. 28, when viewed in the direction facing the support surface 11S, four sub flow paths 15s extend from the main flow path 15m in directions different from each other. Each of the four sub flow paths 15s is open to one of the four side faces 14D. That is, the number of sub flow paths 15s open to each one of the four side faces 14D is 1.

When viewed in the direction facing the support surface 11S, extending directions of the four sub flow paths 15s are preferably equally spaced from each other around the main flow path 15m. With this configuration, the drug can be more evenly distributed and released in the width direction of the projection 18.

In the example shown in Fig. 28, when viewed in the direction facing the support surface 11S, the centers C of openings of sub flow paths 15s that are open to two side faces 14D located on opposite sides of the main flow path 15m are arranged on a straight line K1 that passes through the center E of the region defined by the main flow path 15m.

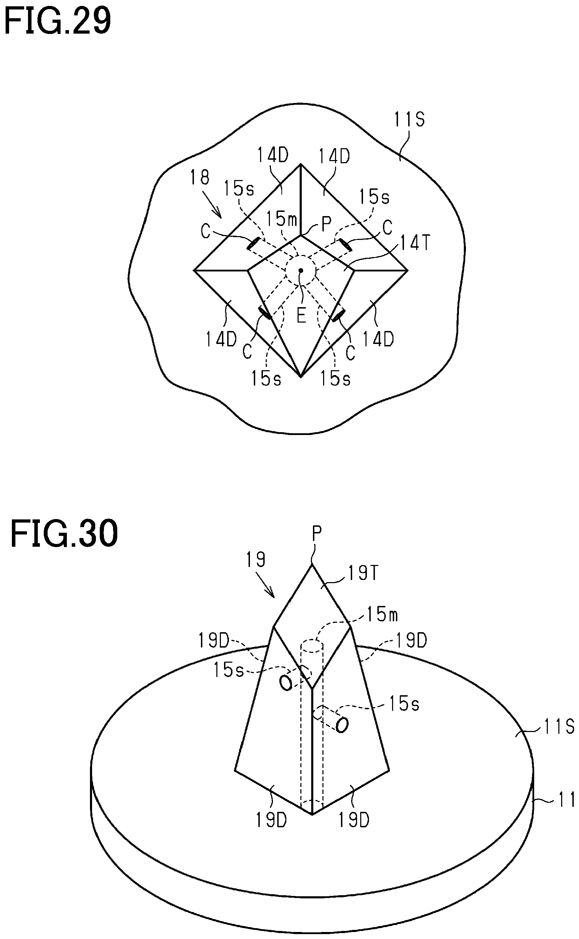

Alternatively, as shown in Fig. 29, the centers C of openings of sub flow paths 15s that are open to two side faces 14D located on opposite sides of the main flow path 15m may not be arranged on the straight line K1 that passes through the center E. With this configuration, since the channels constituting the sub flow paths 15s are prevented from being arranged in one direction when viewed in the direction facing the support surface 11S, it is possible to further prevent a decrease in the strength of the projection 12 against a force applied in a specific direction.

As described above, in the configuration where the sub flow path 15s is open to the side face 14D located closer to the apex P in addition to the side face 14D located farther from the apex P, the drug can be distributed and released in a wider range. Therefore, the drug can be more smoothly administered.

### [Method of Producing Transdermal Administration Device]

Materials and methods for producing the administration section 50 will be described.

For example, the projections 12, 17 and 18 may be made of silicon or metal materials such as stainless steel, titanium, cobalt-chromium alloy, magnesium alloy, and the like. Further, the projections 12, 17 and 18 may be made of resin materials such as commodity plastics, medical grade plastics, and plastics for cosmetic product. Examples of the resin material include polyethylene, polypropylene, polystyrene, polyamide, polycarbonate, cyclic polyolefin, polylactic acid, polyglycolic acid, polycaprolactone, acrylic, urethane resin, aromatic polyether ketones, epoxy resin, and copolymer materials of these resins.

Materials for the base 11 are not specifically limited, and the base 11 may be made of, for example, a material described above as the material for the projections 12, 17 and 18.

The administration section 50 may be formed as a unitary molded product having the base 11 and the projections 12, 17 and 18 integrally formed, or a combination of the base 11 and the projections 12, 17 and 18 which are joined together after they are separately formed, or a combination of a metal material and a resin material. For example, the projections 12, 17 and 18 may be made of a metal and the base 11 may be made of a resin, or vice versa.

When the base 11 and the projections 12, 17 and 18 are separately formed, or when the administration section 50 is formed of a combination of a metal material and a resin material, a sealing agent, adhesive, gasket, O-ring, or the like may also be used as necessary to tightly seal the separate components constituting the administration section 50.

The administration section 50 can be formed by, for example, machining the outer shape of the base 11 and the projections 12, 17 and 18, and then forming the channels as the main flow path 15m and the sub flow paths 15s. Specifically, the outline of the projections 12, 17 and 18, which are composed of the columnar portion 13 and the pyramidal portion 14 is provided by forming a structure having a shape obtained by connecting a cone, pyramid, or frustum to a cylinder or prism, and truncating the structure obliquely relative to the longitudinal direction thereof.

Alternatively, when the administration section 50 is made of a resin material, the administration section 50 can also be formed by a combination of injection molding and machining. For example, after the outer shapes of the base 11 and the projections 12, 17 and 18, and the channel constituting the main flow path 15m, are formed by injection molding, the channels constituting the sub flow paths 15s are formed by machining.

Alternatively, the administration section 50 can also be formed only by injection molding using a combination of a plurality of movable molds.

Examples of the machining technique used for forming the main flow path 15m and the sub flow paths 15s include laser processing.

As described above, according to the second embodiment, the following effects can be achieved.
(2-1) Since the projection includes one main flow path 15m and a plurality of sub flow paths 15s, the drug is distributed and released to a plurality of intradermal sites. Accordingly, the drug more easily spreads into the intradermal layer, rather than when the drug is only released at a single site. Further, since the sub flow paths 15s extend in a direction different from the longitudinal direction of the projection and are open to the peripheral surface of the projection, the skin tissue is less likely to enter the sub flow paths 15s in the puncture process. Therefore, smooth administration of the drug can be performed.
(2-2) Since the height Hs of each sub flow path 15s is different from the heights Hs of other sub flow paths 15s, the plurality of sub flow paths 15s are prevented from being arranged in the same plane extending in the width direction of the projection. As a result, it is possible to prevent the strength of the projection from being significantly reduced at a specific position in the longitudinal direction of the projection, and the projection is less likely to be deformed when it is punctured into the skin. In particular, in the configuration in which the region included in each sub flow path 15s does not include a portion corresponding to the region included in the other sub flow paths 15s in the longitudinal direction of the projection 12, the above effect is enhanced.
(2-3) Since each sub flow path 15s extends from the main flow path 15m in a direction different from the directions in which the other sub flow paths 15s extend from the main flow path 15m when viewed in the direction facing the support surface 11S, the plurality of sub flow paths 15s are prevented from being arranged in the longitudinal direction of the projection. As a result, it is possible to prevent the strength of the projection from being significantly reduced at a specific position in the width direction of the projection, and the projection is less likely to be deformed when it is punctured into the skin. In particular, in the configuration in which the region included in each sub flow path 15s does not include a portion overlapping the region included in the other sub flow paths 15s when viewed in the direction facing the support surface 11S, the above effect is enhanced.
(2-4) Since each sub flow path 15s is open to the side face 14D which is different from that of the other sub flow paths 15s, it is possible to prevent deviation in the positions of the plurality of sub flow paths 15s and an excessive increase of the number of the sub flow paths 15s. Therefore, since it is possible to prevent the strength of the projection from being significantly reduced, the projection is less likely to be deformed when it is punctured into the skin.
(2-5) Since the projection has the pyramidal portion 14 sharpened toward the distal tip end, the projection is easily punctured into the skin. Further, since the projection has the columnar portion 13, the diameter of the hole created by the projection is prevented from expanding near the surface of the skin. As a result, the drug intradermally administered is less likely to leak onto the surface of the skin. Further, since the sub flow path 15s is open to the peripheral surface of the pyramidal portion 14, the effect of preventing drug leakage onto the surface of the skin can be improved.
(2-6) The sum of the sub flow path resistances Sr of all the sub flow paths 15s is equal to the main flow path resistance Mr. Accordingly, for the drug that flows in the main flow path 15m, it is possible to prevent large loss of energy when it flows in the sub flow path 15s. Therefore, the drug can be efficiently delivered from the main flow path 15m to the sub flow paths 15s.

### [Modified Examples]

The second embodiment can be implemented with modifications as described below.
- The shape of the projection is not limited to those described in the above embodiment. For example, as long as the shape of the projection is formed by connecting the bottom of a cone or pyramid to the top of a cylinder or prism and truncating the cone or pyramid obliquely relative to the extending direction thereof, the vertex of the top facet 14T located closest to the base 11 may be located at a position closer to the distal tip end of the projection than the boundary between the cone or pyramid and the cylinder or prism is, or at a position closer to the proximal end of the projection than the boundary is.
   For example, the projection may be formed by truncating a cone or pyramid obliquely relative to the extending direction thereof. Fig. 30 illustrates a projection 19 having a shape formed by truncating a quadrangular prism obliquely relative to the extending direction thereof. In this case, the peripheral surface of the projection 19 is composed of four side faces 19D extending from the support surface 11S in a direction inclined relative to the support surface 11S, and a top facet 19T connected to the four side faces 19D and inclined relative to the support surfaces 11S. Further, when the projection has a shape formed by truncating a cone obliquely relative to the extending direction thereof, the peripheral surface of the projection is composed of a side face, which is a curved surface extending from the support surface 11S, and a top facet connected to the side face and inclined relative to the support surface 11S.
   Further, for example, the projection may have a prismatic shape or a cylindrical shape truncated obliquely relative to the extending direction thereof. Alternatively, the projection may not necessarily have an inclined top facet, and the distal tip end point of the projection may be located at the center of the projection when viewed in a direction facing the support surface 11S. In addition, the top facet of the projection may also be curved, or the peripheral surface of the projection may have a groove or a stepped portion. In short, the only requirement for the projection is that it has a shape able to puncture the skin.
   When the projection has a shape having a dimension in the width direction of the projection gradually increasing from the distal tip end to the proximal end of the projection and thus to the support surface 11S, the strength of the projection near the proximal end is increased. On the other hand, when the projection has the proximal end having a constant dimension in the width direction of the projection, the resistance during puncture of the projection into the skin is reduced compared with the case having a variable dimension in the width direction.
   In the configuration in which the heights Hs of the plurality of sub flow paths 15s are different from each other, a region included in any one of the sub flow paths 15s may include a portion overlapping a portion included in another sub flow path 15s in the longitudinal direction of the projection 12. Further, in the configuration in which the extending direction of the plurality of sub flow paths 15s are different from each other when viewed in the direction facing the support surface 11S, a region included in any one of the sub flow paths 15s may include a portion overlapping a portion included in another sub flow path 15s.
   As long as the projection includes the plurality of sub flow paths 15s that are open to the peripheral surface of the projection, the positions of the openings of the sub flow paths 15s and the extending directions of the sub flow paths 15s are not limited.
   For example, when two sub flow paths 15s are provided, the sub flow paths 15s may be open to each of the two side faces 14D closer to the apex P, or may be open to one of the side faces 14D closer to the apex P and one of the side faces 14D farther from the apex P. Further, when three or four sub flow paths 15s are provided, the arrangement of the openings may be different from the arrangement described in the above embodiments. In addition, two or more sub flow paths 15s may be open to one of the side faces 14D of the projection, or may be open to the top facet 14T or the side face 13D of the columnar portion 13, or at the boundary between the adjacent surfaces that constitute the peripheral surface of the projection.
   Moreover, the plurality of sub flow paths 15s may be arranged in the longitudinal direction or the width direction of the projection. That is, some of the plurality of sub flow paths 15s may be located at the same height Hs, or may extend from the main flow path 15m in the same direction as viewed when the direction facing the support surface 11S.
   Further, the sub flow path 15s may extend in a direction different from the width direction of the projection, in other words, may extend in the direction inclined relative to the support surface 11S. Moreover, the sub flow path 15s located farthest from the support surface 11S may extend from a position at the same height as the distal end of the main flow path 15m. Alternatively, at the connection between the sub flow path 15s located farthest from the support surface 11S and the main flow path 15m, the distal end of the sub flow path 15s may be located closer to the distal tip end of the projection 12 than to the distal end of the main flow path 15m.
   The effect described in the above (2-1) can be obtained as long as the projection includes the plurality of sub flow paths 15s that are open to the peripheral surface of the projection.
   The region defined by each of the main flow path 15m and the sub flow path 15s in a cross-section perpendicular to the extending direction of the flow path is not limited to a circular shape, and may be, for example, a rectangular shape. Further, the shape of the region defined by the main flow path 15m in the above cross-section may be different from the shape of the region defined by the sub flow path 15s in the above cross-section. The sub flow path area Ss may be different among the sub flow paths 15s, or the sum of the sub flow path resistances Sr of all the sub flow paths 15s may be different from the main flow path area Ms. Further, the sum of the sub flow path resistances Sr of all the sub flow paths 15s may be different from the main flow path resistance Mr.
- Usage of the administration section 50 is not limited to being mounted on the syringe barrel 60. A drug may be supplied into the main flow path 15m of the projection by using a tool other than the syringe barrel 60. Further, the projection may be separated from the base 11 after administration of the drug and left in the skin of the administration target.
- The target for drug administration using a transdermal administration device is not limited to a human, but may also be another animal. Further, the configurations of the above embodiments and the modified examples can be appropriately combined for use.

### [Examples]

The transdermal administration device of the second embodiment will now be described by using specific examples and comparative examples.

### <Example 2-1>

A polycarbonate was used as a material for the administration section. A plate-shaped base and a structure having a shape formed by connecting a quadrangular pyramid to the top of a quadrangular prism having a square bottom, and truncating the quadrangular pyramid obliquely relative to the extending direction, the structure including a channel therein as a main flow path, were produced by injection molding. Further, a projection was produced by forming two channels as sub flow paths in the structure by laser processing. Thus, a transdermal administration device of Example 2-1 having only an administration section was obtained.

The projection of the administration section of Example 2-1 had a shape as shown in Figs. 19 to 21, wherein a length Lt of the projection was 750 µm, and a length Lc of the columnar portion was 100 µm. Further, an inclination of the side face of the pyramidal portion relative to the support surface was 80°. A height Hm of the main flow path was 600 µm, and heights Hs of two sub flow paths were 460 µm and 510 µm in ascending order. The region defined by the main flow path in a cross-section perpendicular to the extending direction of the main flow path had a circular shape, and the diameter thereof was 100 µm. The region defined by each of the two sub flow paths in a cross-section perpendicular to the extending direction of the sub flow path was a circular shape, and the diameter thereof was 70 µm.

### <Example 2-2>

An administration section having the same shape as in Example 2-1 except for a configuration of the sub flow path was obtained by the same process as in Example 2-1. Thus, a transdermal administration device of Example 2-2 was obtained.

The projection of the administration section of Example 2-2 had three sub flow paths, and the projection of Example 2-2 had a shape shown in Figs. 23 to 25. Heights Hs of the three sub flow paths were 350 µm, 430 µm, and 510 µm in ascending order. The sub flow path having the largest height Hs was open at the boundary between two side faces located closer to the apex P of the projection. When viewed in the direction facing the support surface, three sub flow paths were disposed clockwise from a position on one of the side faces located farther from the apex P of the projection, in ascending order of their heights Hs. The region defined by each of the three sub flow paths in a cross-section perpendicular to the extending direction of the sub flow path was a circular shape, and the diameter thereof was 58 µm.

### <Example 2-3>

An administration section having the same shape as in Example 2-1 except for a configuration of the sub flow path was obtained by the same process as in Example 2-1. Thus, a transdermal administration device of Example 2-3 was obtained.

The projection of the administration section of Example 2-3 had four sub flow paths, and the projection of Example 2-3 had a shape shown in Figs. 26 to 28. Heights Hs of the four sub flow paths were 350 µm, 400 µm, 450 µm, and 510 µm in ascending order. Each of the sub flow path having the smallest height Hs and the sub flow path having the second smallest height Hs were open to each one of two side faces located farther from the apex P of the projection. Each of the sub flow path having the largest height Hs and the sub flow path having the second largest height Hs were open to each one of two side faces located farther from the apex P of the projection. When viewed in the direction facing the support surface, four sub flow paths were disposed clockwise from a position on one of the side faces located farther from the apex P of the projection, in ascending order of their heights Hs. The region defined by each of the four sub flow paths in a cross-section perpendicular to the extending direction of the sub flow path was a circular shape, and the diameter thereof is 50 µm.

### <Comparative Example 2-1>

An administration section having the same shape as in Example 2-1 except for a configuration of the sub flow path was obtained by the same process as in Example 2-1. Thus, a transdermal administration device of Comparative Example 2-1 was obtained.

In the projection of the administration section of Comparative Example 2-1, one sub flow path was formed, and the height Hs of the sub flow path was 450 µm. The sub flow path was open at the boundary between two side faces located closer to the apex P of the projection. The region defined by the sub flow path in a cross-section perpendicular to the extending direction of the sub flow path was a circular shape, and the diameter thereof was 100 µm.

### <Comparative Example 2-2>

An administration section having the same shape as in Example 2-1 except for a configuration of the flow path was obtained by the same process as in Example 2-1. Thus, a transdermal administration device of Comparative Example 2-2 was obtained.

The administration section of Comparative Example 2-2 did not include a sub flow path. Instead of the main flow path having a closed distal end, a through hole that penetrates the projection and the base from the proximal end to the distal tip end of the projection was formed. Such a through hole was formed by adjusting the length of the channel inside the structure formed by injection molding in the production process of Example 2-1. The region defined by the through hole in a cross-section perpendicular to the extending direction of the through hole had a circular shape, and the diameter thereof was 100 µm.

### [Evaluation of Liquid Release Capability]

For the transdermal administration devices of Examples 2-1 to 2-3, and Comparative Example 2-1 in which a sub flow path is formed, liquid release capability was evaluated. Each transdermal administration device was attached to a 1mL tuberculin syringe barrel filled with pure water. The pure water was kept pressed with a 0.2 MPa force so that the water was released into the atmosphere from the sub flow path of the projection, and the time required to complete release of 1000 µL of water was measured. The measurement was performed three times for each of the examples and the comparative example. Pressing the pure water was performed by inserting a tube connected to a pressure gauge into an outer cylinder of the syringe barrel as a substitute for a plunger, and supplying a gas into the tube until the pressure inside the tube reached 0.2 MPa.

Table 1 shows the number of the sub flow paths, measurements of the time required to complete release of the above pure water, and the average of these times for the transdermal administration devices of Examples 2-1 to 2-3, and Comparative Example 2-1. Further, the average is shown by rounding off the second decimal place.

**[Table 1]**

| | Number of sub flow paths | Release time (s) | | | |
|---|---|---|---|---|---|
| | | n=1 | n=2 | n=3 | Average |
| Example 2-1 | 2 | 22 | 22 | 22 | 22.0 |
| Example 2-2 | 3 | 21 | 25 | 25 | 23.7 |
| Example 2-3 | 4 | 22 | 23 | 23 | 22.7 |
| Comparative Example 2-1 | 1 | 22 | 21 | 20 | 21.0 |

As seen from Table 1, in each of Examples 2-1 to 2-3 and Comparative Example 2-1, the time required to complete release of 1000 µL pure water under a pressure of 0.2 MPa was approximately 22 seconds, and was substantially the same. That is, it is suggested that main flow path and the sub flow path in the transdermal administration devices of Examples 2-1 to 2-3 and Comparative Example 2-1 are configured such that the transdermal administration devices can release the same amount of liquid in the same period of time under the same pressure. Further, although the configuration of flow path of Comparative Example 2-1 differs from that of Comparative Example 2-2 in that the flow path is bent in Comparative Example 2-1 and the flow path extends in one direction in Comparative Example 2-2, the diameter of flow path of Comparative Example 2-1 is the same as that of Comparative Example 2-2. Accordingly, it is anticipated that the time required to complete release of 1000 µL pure water in Comparative Example 2-2 would be similar to that of Comparative Example 2-1.

### [Evaluation of Administration Amount of Drug]

Administration amount of drug was evaluated by using the transdermal administration devices of Examples 2-1 to 2-3 and Comparative Examples 2-1 and 2-2.

Each transdermal administration device was attached to a syringe barrel, which was filled with saline solution as a drug, and the projection was punctured into a skin taken from a 12-week-old Wistar rat. Then, the drug was pressed toward the projection by a force of 0.2 MPa. Pressing the drug was performed by inserting a tube connected to a pressure gauge into an outer cylinder of the syringe barrel as a substitute for a plunger, and supplying a gas into the tube until the pressure inside the tube reached 0.2 MPa. The drug was continuously pressed for 300 seconds while observing the skin of the Wistar rat, and the amount of the drug intradermally administered, that is, a maximum amount of the drug that was successfully injected without leaking onto the skin surface or the hypodermis was measured. The upper limit of the administration amount of drug was set to 600 µL, and administration was terminated when the administration amount exceeded 600 µL.

Table 2 shows the number of the sub flow paths and the measurement results of the amount of the drug intradermally administered for the transdermal administration devices of the respective examples and comparative examples. In the test using the transdermal administration device of Comparative Example 2-2, leakage of the drug was observed on the surface of the extracted skin on a side opposite to that to which the drug was administered. In Comparative Example 2-2, the total administration amount of drug was 600 µL, and the amount of drug intradermally administered was calculated as 100 µL by estimating the amount of drug leaked into the hypodermis.

**[Table 2]**

| | Number of sub flow paths | Administration amount (µL) |
|---|---|---|
| Example 2-1 | 2 | 200 |
| Example 2-2 | 3 | 600 |
| Example 2-3 | 4 | 600 |
| Comparative Example 2-1 | 1 | 100 |
| Comparative Example 2-2 | - (Through hole) | 100 |

As seen from Table 2, in each of Examples 2-1 to 2-3, a larger amount of drug can be administered than in Comparative Examples 2-1 and 2-2. That is, in the example in which the plurality of sub flow paths are provided, the drug more easily spreads into the intradermal layer than in the comparative examples. Accordingly, it is suggested that a larger amount of drug can be intradermally injected since it is possible to avoid a failure in injection of the drug due to the pressure of 0.2 MPa being insufficient and leakage of the drug onto the skin surface or into the hypodermis. Accordingly, it was found that smooth administration of the drug can be performed in the example in which a plurality of sub flow paths are provided, compared with the comparative examples.

Further, a larger amount of drug can be administered in Examples 2-2 and 2-3 than in Example 2-1. Therefore, it was found that smooth administration of the drug can be performed in the configuration in which the number of sub flow paths is three or more, and the sub flow paths are open to the side faces located closer to the apex P as well as the side faces located farther from the apex P compared with the configuration in which the number of sub flow paths is two, and the sub flow paths are open only to the side faces located farther from the apex P.

### <Example 2-4>

An administration section having the same shape as in Example 2-1 except for a configuration of the sub flow path was obtained by the same process as in Example 2-1. Thus, a transdermal administration device of Example 2-4 was obtained.

In the projection of the administration section of Example 2-4, two sub flow paths were arranged in the longitudinal direction of the projection. That is, when viewed in the direction facing the support surface, the two sub flow paths extended in the same direction from the main flow path. Heights Hs of the two sub flow paths were 360 µm and 510 µm in ascending order. The two sub flow paths were open at the boundary between two side faces located closer to the apex P of the projection. The region defined by each of the two sub flow paths in a cross-section perpendicular to the extending direction of the sub flow path was a circular shape, and the diameter thereof was 70 µm.

### <Example 2-5>

An administration section having the same shape as in Example 2-1 except for a configuration of the sub flow path was obtained by the same process as in Example 2-1. Thus, a transdermal administration device of Example 2-5 was obtained.

In the projection of the administration section of Example 2-5, three sub flow paths were provided at the same height Hs. That is, the three sub flow paths were arranged in a plane parallel to the width direction of the projection. Heights Hs of the three sub flow paths were 400 µm. The three sub flow paths were open to each of the two side faces located farther from the apex P of the projection and at the boundary between two side faces located closer to the apex P of the projection. The region defined by each of the three sub flow paths in a cross-section perpendicular to the extending direction of the sub flow path was a circular shape, and the diameter thereof was 70 µm.

### [Evaluation of Strength of Projection]

For the transdermal administration devices of Examples 2-1 to 2-5 and Comparative Example 2-1, in which the sub flow path was formed, the strength of the projection was evaluated.

Each transdermal administration device was attached to a syringe barrel, and the projection was punctured into the skin taken from a 12-week-old Wistar rat. Then, the transdermal administration device was pulled out from the skin. The transdermal administration device was observed with a stereomicroscope before and after puncture into the skin to check presence or absence of a change in shape.

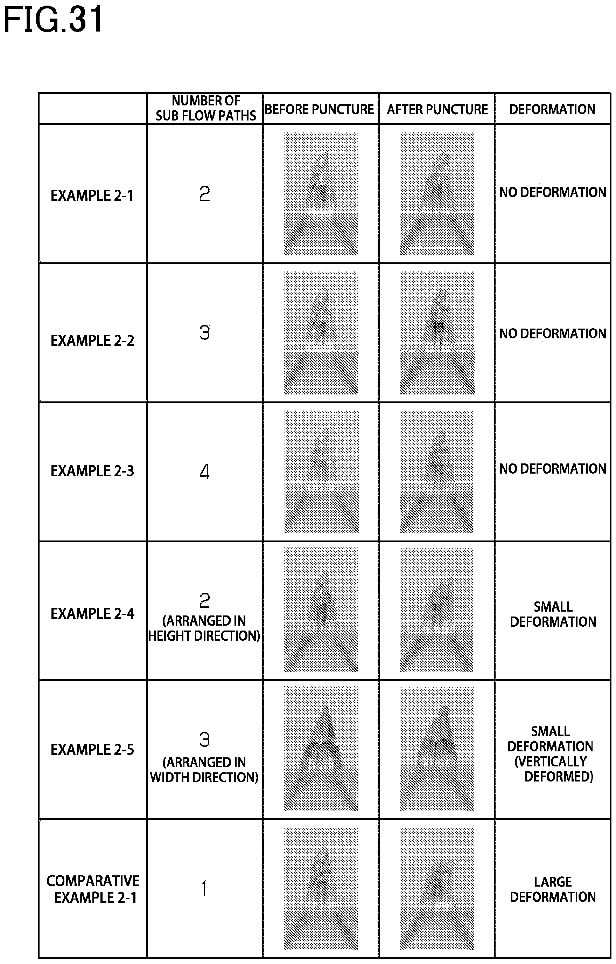

Fig. 31 shows an evaluation result of the number of sub flow paths, images of the projection taken before and after the puncture, and presence or absence of a change in shape of the projection for the transdermal administration devices of Examples 2-1 to 2-5 and Comparative Example 2-1.

As shown in Fig. 31, the projection of the comparative example more deforms more significantly compared to that in the inventive examples. That is, when the sums of all the sub flow path areas Ss are equal, it was found that the strength of the projection was higher in the configuration having a plurality of sub flow paths than in the configuration having one sub flow path.

Moreover, among the examples, deformation of the projection was not observed in Examples 2-1 to 2-3, while deformation of the projection was observed in Examples 2-4 and 2-5. That is, it was found that the strength of the projection was higher and the projection was less likely to deform during puncture in the configurations in which the plurality of sub flow paths were not arranged in the longitudinal direction or in the width direction of the projection.

### (Third Embodiment)

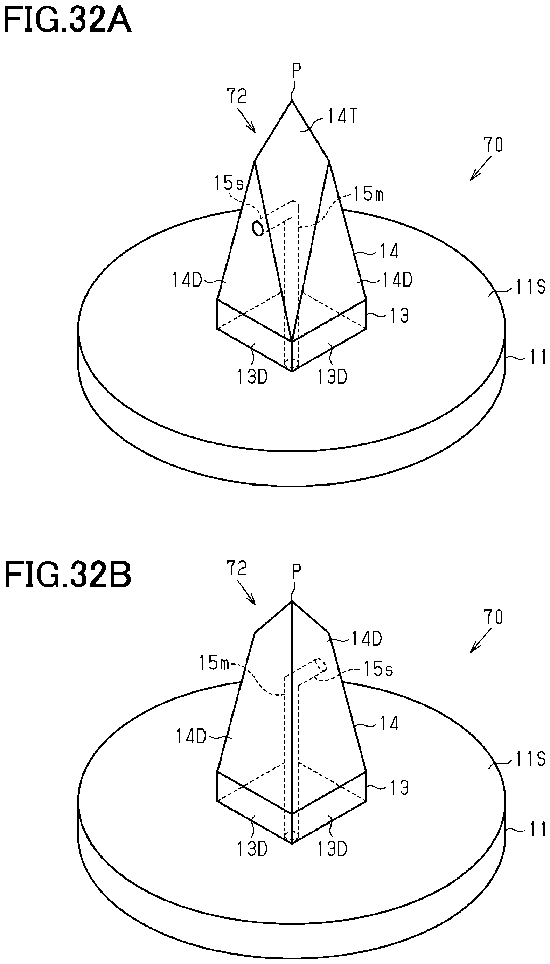

With reference to Figs. 32 to 36, a third embodiment of a transdermal administration device will be described.

### [Problem to be Solved by Third Embodiment]

In the administration method using a conventional microneedle device, the distal tip end of the projection is located in the intradermal layer of the skin during drug administration, in contrast to subcutaneous injection by which the distal tip end of the needle reaches the hypodermis. The intradermal layer has higher tissue density than the hypodermis, and the through hole of the projection has a relatively small diameter compared with that of typical injection needles, and further, skin tissue may occasionally enter the through hole. For these reasons, a smooth flow of the drug from the through hole into the intradermal layer may be disturbed. Therefore, from the viewpoint of smooth administration of a drug into the intradermal layer, there is still room for improvement in the configuration of the flow path for a drug provided in the projection.

The third embodiment is directed to provide a transdermal administration device that enables smooth administration of a drug.

### [Overall Configuration of Transdermal Administration Device]

The transdermal administration device of the third embodiment may include at least an administration section 70. In the third embodiment, the administration section 70 is a microneedle device.

As shown in Figs. 32A and 32B, the administration section 70 includes a base 11 having a support surface 11S, and a projection 72 protruding from the support surface 11S.

The support surface 11S supports a proximal end of the projection 72. The shape of the support surface 11S is not specifically limited, and may have a circular or polygonal shape. The base 11 may have a flat plate shape as shown in Figs. 32A and 32B, or may have a tubular shape extending from the support surface 11S in a direction opposite to the extending direction of the projection 72. The base 11 may include a structure such as groove or boss on the outer periphery of the base 11 or the like for connecting the administration section 70 to a device for supplying a liquid drug to the administration section 70.

The projection 72 includes a columnar portion 13 having a cylindrical or prismatic shape extending from the support surface 11S, and a pyramidal portion 14 having a conical or pyramid shape extending from the top of the columnar portion 13 and truncated obliquely relative to the extending direction of the conical or pyramid shape.

Specifically, the columnar portion 13 has a quadrangular prism shape, and has four side faces 13D extending from a square bottom defined on the support surface 11S. The side face 13D is perpendicular to the support surface 11S. Further, the pyramidal portion 14 has a shape formed by truncating a quadrangular pyramid obliquely relative to the extending direction thereof. Accordingly, the pyramidal portion 14 has four side faces 14D and one top facet 14T, whose edges are shared with each of the side faces 14D.

The side faces 14D are inclined relative to the support surface 11S, and each side face 14D shares one edge with one side face 13D. The top facet 14T is also inclined relative to the support surface 11S, and all the edges included in the top facet 14T are also inclined relative to the support surface 11S. As shown in the example in Figs. 32A and 32B, among the vertices of the top facet 14T, the vertex located closest to the base 11, that is, the vertex located closest to the proximal end of the projection 72, is located at the end in contact with the proximal end of the pyramidal portion 14, that is, the end in contact with the columnar portion 13. In this configuration, each side face 14D which shares a vertex of the top facet 14T located closest to the base 11 with the top facet 14T has a triangular shape.

The top facet 14T has a shape symmetrical with respect to a straight line connecting the vertex closest to the base 11 and the vertex farthest from the base 11, and the vertex farthest from the base 11 constitutes an apex P, which is the apex of the projection 72.

The peripheral surface of the projection 72 is composed of the above four side faces 13D, four side faces 14D, and the top facet 14T. A length of the projection 72 from the support surface 11S is largest at the apex P.

The projection 72 includes one main flow path 15m which extends therein from the proximal end toward the distal tip end of the projection 72, and one sub flow path which extends from the main flow path 15m toward the peripheral surface of the projection 72. The main flow path 15m communicates with the sub flow path 15s, and both the main flow path 15m and the sub flow path 15s define a space therein for allowing a fluid to flow in the projection 72. The main flow path 15m penetrates the support surface 11S and communicates with the outside of the administration section 70, while the sub flow path 15s is open to the peripheral surface of the projection 72.

The administration section 70 may include a single projection 72 or a plurality of projections 72. When the administration section 70 includes a single projection 72, the projection 72 is preferably located at a center of the support surface 11S. Further, when the administration section 70 includes a plurality of projections 72, the plurality of projections 72 are arranged, for example, in a grid, circular, or coaxial pattern on the support surface 11S.

### [Detailed Configuration of Flow Paths]

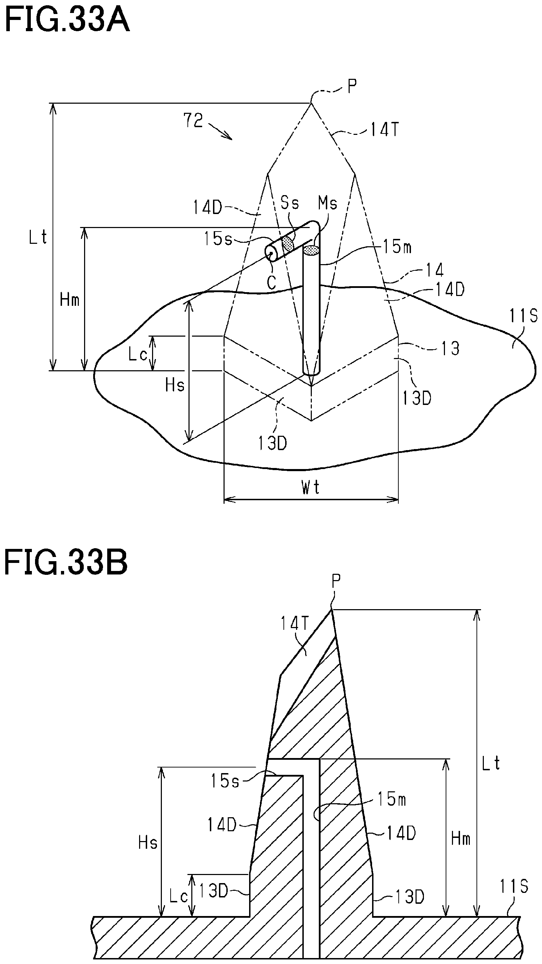

With reference to Figs. 33 to 35, a detailed configuration of the main flow path 15m and the sub flow path 15s will be described. Fig. 33A is a view in which an outline of the main flow path 15m and the sub flow path 15s are indicated by the solid line, and an outline of the projection 72 is indicated by the double dotted and dashed line. Fig. 33B is a view illustrating a cross-section of the projection 72 taken along the extending direction of the sub flow path 15s.

As shown in Figs. 33A and 33B, the main flow path 15m is located in a center part of the projection 72, and extends in a longitudinal direction of the projection 72, that is, in a direction perpendicular to the support surface 11S. In the extending direction of the main flow path 15m, one of the ends of the main flow path 15m adjacent to the proximal end of the projection 72 is an open end. For example, the main flow path 15m penetrates the base 11 and is open to a surface of the base 11 on a side opposite to the support surface 11S. On the other hand, in the extending direction of the main flow path 15m, the other of the ends of the main flow path 15m, which is close to the distal tip end of the projection 72, is closed. That is, the end of the main flow path 15m close to the distal tip end of the projection 72 does not reach the top facet 14T of the projection 12.

The sub flow path 15s extends in a width direction of the projection 72, that is, in a direction parallel to the support surface 11S. One of the ends of the sub flow path 15s in the extending direction is connected to the main flow path 15m, and the other of the ends is open to the side face 14D of pyramidal portion 14 of the projection 72. The side face 13D of the top facet 14T and the columnar portion 13 do not include an opening which is an end of the flow path.

The main flow path 15m and the sub flow path 15s constitute flow paths in the projection 72 for a fluid flowing via the opening end of the main flow path 15m toward the openings of the sub flow path 15s, and the flow path is bent at a connecting section between the main flow path 15m and the sub flow path 15s.

A region defined by each of the main flow path 15m and the sub flow path 15s in a cross-section perpendicular to the extending direction of the flow path is a circular shape. In a cross-section perpendicular to the extending direction of the main flow path 15m, an area of the region defined by the main flow path 15m is a main flow path area Ms, and the main flow path area Ms is constant in each main flow path 15m. In the sub flow path 15s, an area of the region defined by the sub flow path 15s in a cross-section perpendicular to the extending direction of the sub flow path 15s is a sub flow path area Ss, and the sub flow path area Ss is constant in one sub flow path 15s.

The sub flow path area Ss is 300 µm² or more, and preferably 700 µm² or more. Further, the sub flow path area Ss is preferably 8000 µm² or less, and more preferably 2000 µm² or less.

The main flow path area Ms is preferably 3000 µm² or more and 70000 µm² or less. The main flow path area Ms and the sub flow path area Ss may be the same or different from each other.

A length Lt of the projection 72 is a length in the longitudinal direction of the projection 72, that is, from the support surface 11S to the apex P of the projection 72 in a direction perpendicular to the support surface 11S. The length Lt of the projection 72 is preferably a length that penetrates the stratum corneum, which is the outermost layer of the skin, but does not reach the hypodermis, and specifically, preferably in the range of 200 µm or more and 2000 µm or less.

Further, a length Lc of the columnar portion 13 is a length from the support surface 11S to the top of the columnar portion 13 in the longitudinal direction of the projection 72, and the length Lc of the columnar portion 13 is preferably 1/20 or more and 1/2 or less of the length Lt of the projection 72.

A width Wt of the projection 72 is a maximum length of the projection 72 in the width direction of the projection 72, that is, in a direction parallel to the support surface 11S. That is, the width Wt of the projection 72 is a length of a diagonal of a square formed by the bottom of the projection 72 defined on the support surface 11S. A width Wt of the projection 72 is preferably in the range of 150 µm or more and 1000 µm or less.

A height Hm of the main flow path 15m is a length of the main flow path 15m from the support surface 11S in the longitudinal direction of the projection 72, that is, a length of the main flow path 15m from the support surface 11S to the distal end of the main flow path 15m. The height Hm of the main flow path 15m is smaller than the length Lt of the projection 72.

A height Hs of the sub flow path 15s is a length from the support surface 11S to a center C of the opening of the sub flow path 15s in the longitudinal direction of the projection 72. The center C of the opening is the center of gravity of the figure formed by a region defined by the opening, and is located on a straight line passing through the center of gravity of the region defined by the sub flow path 15s in the cross-section perpendicular to the extending direction of the sub flow path 15s. For example, in the case where the sub flow path 15s is circular in each cross-section and the sub flow path 15s is a cylinder, the center C of the opening of the sub flow path 15s is located on the straight line passing through the center of the circle, that is, on the center axis of the cylinder.

The height Hs of the sub flow path 15s is preferably 100 µm or more. In other words, the center C of the sub flow path 15s is preferably separated from the support surface 11S by 100 µm or more. When the height Hs of the sub flow path 15s is 100 µm or more, the drug supplied to the projection 72 is prevented from flowing out of the projection 72 at a position near the support surface 11S, that is, a position near the surface of the skin. Accordingly, the drug intended to be administered into the intradermal layer is prevented from leaking onto the skin. Further, the height Hs of the sub flow path 15s is preferably 1500 µm or less.

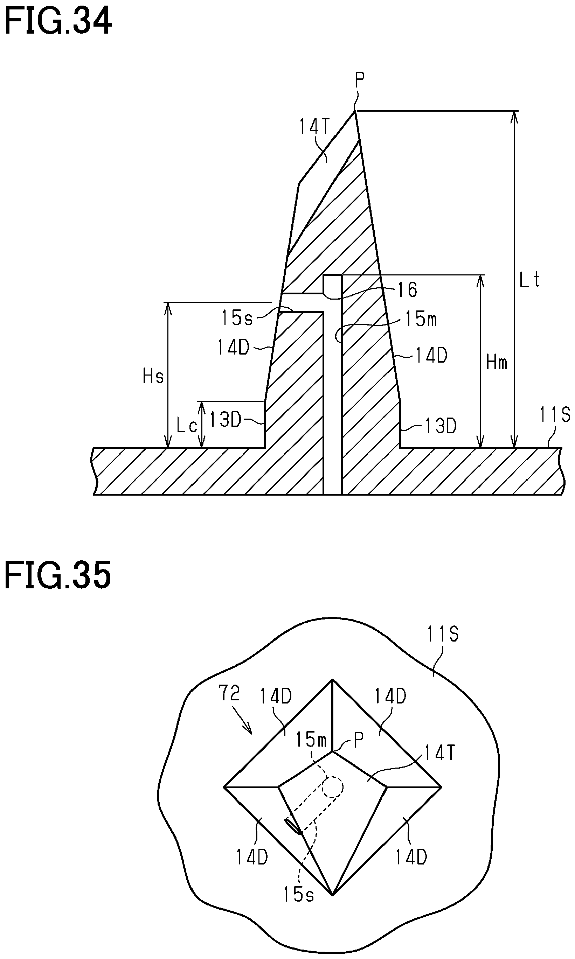

As shown in Fig. 33B, the sub flow path 15s may extend from a position at the same height as the distal end of the main flow path 15m. Alternatively, as shown in Fig. 34, in the sub flow path 15s, a distance from an end connected to the main flow path 15m to the proximal end of the projection 72 may be smaller than a distance from the distal end of the main flow path 15m to the proximal end of the projection 12. In either case, the height Hs of the sub flow path 15s is smaller than the height Hm of the main flow path 15m.

In the configuration shown in Fig. 33B, at the connecting section between the sub flow path 15s and the main flow path 15m, the position of the distal end of the main flow path 15m matches the position of the end of the sub flow path 15s which is closer to the distal tip end of the projection 72 in the longitudinal direction of the projection 72.

On the other hand, in the configuration shown in Fig. 34, the main flow path 15m extends toward the distal tip end of the projection 72 beyond the sub flow path 15s in the longitudinal direction of the projection 72, and the stepped portion 16 is formed at the connecting section between the sub flow path 15s and the main flow path 15m. In the configuration shown in Fig. 34, high precision is not required for positioning of the main flow path 15m and the sub flow path 15s compared with the configuration shown in Fig. 33B, when the administration section 70 is produced by forming the main flow path 15m and then forming the sub flow path 15s.

As shown in Fig. 35, when viewed in the direction facing the support surface 11S, the apex P of the projection 72 is located on an edge of the top facet 14T. The side face 14D to which the sub flow path 15s is open is a side face 14D which does not include the apex P of the projection 72 and shares an edge of the top facet 14T located on the lower side of the slope, that is, the edge close to the proximal end of the projection 72, with the top facet 14T. In other words, when four side faces 14D are divided into two side faces 14D closer to the apex P and two side faces 14D farther from the apex P, the sub flow path 15s is open to either of the side faces 14D farther from the apex P. The side face 14D closer to the apex P is the side face 14D that shares an edge of the top facet 14T which includes the apex P with the top facet 14T, and the side face 14D farther from the apex P is the side face 14D that shares an edge of the top facet 14T which does not include the apex P with the top facet 14T.

In a configuration in which the apex P is located on the edge of the top facet 14T, when the projection 72 is punctured into the skin, the projection 72 undergoes a force pressing against the top facet 14T, that is, a force that causes the projection 72 to be inclined into a side where the apex P is located rather than toward the center of the projection 72. Therefore, in the configuration in which the sub flow path 15s is open to the side face 14D located farther from the apex P, compared with a configuration in which the sub flow path 15s is open to the side face 14D located closer to the apex P, the projection 72 has an increased strength against the force that causes the projection 72 to be inclined to a side where the apex P is located.

### [Effects]

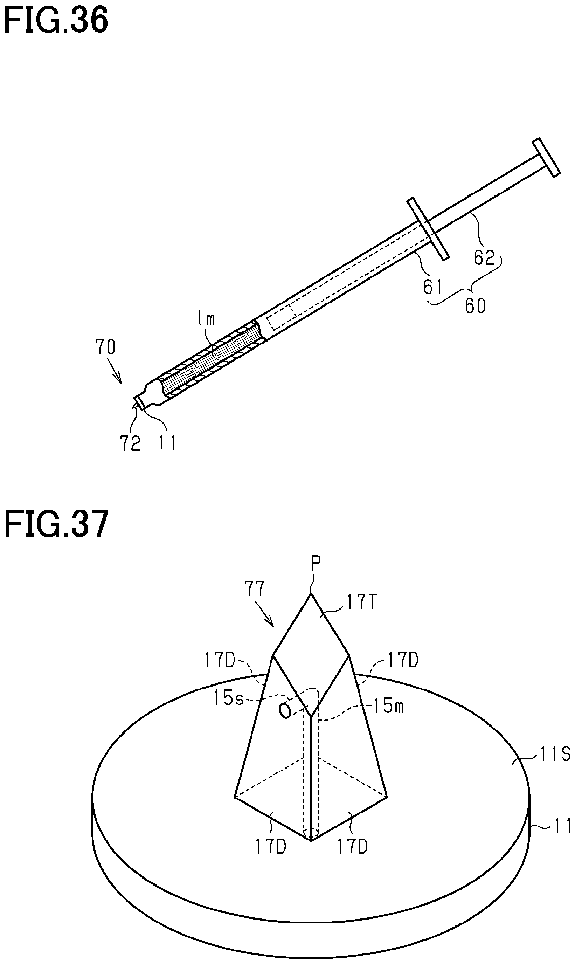

With reference to Fig. 36, effects of the administration section 70 of the present embodiment will be described.

As shown in Fig. 36, the administration section 70 is mounted on an end of an outer cylinder 61 of a syringe barrel 60. In administration of a liquid drug, the administration section 70 is pressed against the skin of administration target of the liquid drug to puncture the skin using the projection 72. Then, while the projection 72 is punctured into the skin, the plunger 62 is pushed into the administration section 70. As the plunger 62 is pushed, a liquid drug lm loaded in the outer cylinder 61 is supplied into the main flow path 15m of the projection 72, and the drug lm is further fed from the main flow path 15m into the sub flow path 15s. Then, the drug lm flows out from the opening on the side face 14D of the projection 72 and flows into the intradermal layer.

While the drug is released in the longitudinal direction of the projection in the conventional configuration in which the end of the through hole is open in the vicinity of the distal tip end of the projection, the drug is released in the width direction of the projection 72 from the projection 72 of the present embodiment. While the projection 72 is inserted into the skin, the intradermal tissues spread in the width direction of the projection 72 rather than the longitudinal direction. Accordingly, with the above configuration, the drug can easily spread into the intradermal layer without leaking into the hypodermis compared with the conventional configuration.

Further, the projection 72 is inserted into the skin from the apex P in the longitudinal direction. Therefore, compared with the conventional configuration in which the end of the through hole is open in the vicinity of the distal tip end of the projection, the skin tissue is less likely to enter the drug flow path in the puncture process in the configuration of the present embodiment in which the end of the sub flow path 15s which extends in directions different from the longitudinal direction of the projection 72 is open to the peripheral surface of the projection 72.

When the sub flow path area Ss is small, and thus the outlet for a drug flowing out from the projection 72 is small, the drug is not likely to smoothly flow from the sub flow path 15s into the intradermal layer. The drug can be smoothly released from the sub flow path 15s into the intradermal layer when the sub flow path area Ss is 300 µm² or more. The drug is particularly smoothly released from the sub flow path 15s into the intradermal layer when the sub flow path area Ss is 700 µm² or more.

Furthermore, when the sub flow path area Ss is 8000 µm² or less, appropriate administration of the drug into the skin of a human, and even of a small animal as an administration target of a test, is possible since the size of the opening of the sub flow path 15s relative to the thickness of the skin is not too large.

Thus, according to the administration section 70 of the present embodiment, smooth administration of the drug can be performed. As a result, it is possible to prevent an increase in the burden on the person administering the drug and the recipient of the drug since the force required to press the drug and the time required to administer the drug are prevented from increasing. Further, since leakage of the drug to the surface of the skin or to the hypodermis due to the limitation on the amount of the drug that can spread into the intradermal layer is reduced, it is possible to prevent a decrease in the amount of the drug intradermally administered due to the drug leaking to a site different from the intended position.

In addition, the projection 72 is easily punctured into the skin since the projection 72 has the pyramidal portion 14 sharpened toward the distal tip end. Further, since the projection 72 has the columnar portion 13, the diameter of the hole created by the projection is prevented from expanding near the surface of the skin, compared with the configuration in which the projection is composed of only the pyramidal portion 14, the side face inclined relative to the support surface 11S extends to the support surface 11S. As a result, the drug intradermally administered is less likely to leak onto the surface of the skin. In particular, since the sub flow path 15s is open to the side face 14D of the pyramidal portion 14, and the side face 13D of the columnar portion 13 does not include the opening, which is the end of the sub flow path 15s, the effect of preventing drug leakage onto the surface of the skin can be improved.

### [Method of Producing Transdermal Administration Device]

Materials and methods for producing the administration section 70 will be described.

Materials for forming the projection 72 are not specifically limited, and the projection 72 may be made of silicon, or metal materials such as stainless steel, titanium, cobalt-chromium alloy, and magnesium alloy. Further, the projection 72 may be made of resin materials such as commodity plastics, medical grade plastics, and plastics for cosmetic product. Examples of the resin material include polyethylene, polypropylene, polystyrene, polyamide, polycarbonate, cyclic polyolefin, polylactic acid, polyglycolic acid, polycaprolactone, acrylic, urethane resin, aromatic polyether ketones, epoxy resin, and copolymer materials of these resins.

Materials for the base 11 are not specifically limited, and the base 11 may be made of, for example, a material described above as the material for the projection 72.

The administration section 70 may be formed as a unitary molded product having the base 11 and the projection 72 integrally formed, or a combination of the base 11 and the projection 72 which are joined together after they are separately formed, or a combination of a metal material and a resin material. For example, the projection 72 may be made of a metal and the base 11 may be made of a resin, or vice versa.

When the base 11 and the projection 72 are separately formed, or when the administration section 70 is formed of a combination of a metal material and a resin material, a sealing agent, adhesive, gasket, O-ring, or the like may also be used as necessary to tightly seal the separate components constituting the administration section 70.

The administration section 70 can be formed by, for example, machining the outer shape of the base 11 and the projection 72, and then forming the channels as the main flow path 15m and the sub flow path 15s. Specifically, the outline of the projection 72, which is composed of the columnar portion 13 and the pyramidal portion 14 is provided by forming a structure having a shape obtained by connecting a cone, pyramid, or frustum to a cylinder or prism, and truncating the structure obliquely relative to the longitudinal direction thereof.

Alternatively, when the administration section 70 is made of a resin material, the administration section 70 can also be formed by combination of injection molding and machining. For example, after the outer shapes of the base 11 and the projection 72, and the channel constituting the main flow path 15m are formed by injection molding, the channel constituting the sub flow path 15s is formed by machining.

Alternatively, the administration section 70 can also be formed only by injection molding by combination of a plurality of movable molds.

Examples of the machining technique used for forming the main flow path 15m and the sub flow path 15s include laser processing.

As described above, according to the third embodiment, the following effects can be achieved.
(3-1) Since the projection 72 includes the sub flow path 15s that extends from the main flow path 15m and is open to the peripheral surface of the projection 72, the drug can easily spread into the intradermal layer without leaking into the hypodermis. Further, when the sub flow path area Ss in the sub flow path 15s is 300 µm² or more, the drug can be smoothly released from the sub flow path 15s into the intradermal layer. Still further, when the sub flow path area Ss is 700 µm² or more, the drug can be more smoothly released from the sub flow path 15s into the intradermal layer.
(3-2) When the sub flow path area Ss is 8000 µm² or less, appropriate administration of the drug into the intradermal layer is possible since the size of the opening of the sub flow path 15s relative to the thickness of the skin is not too large.
(3-3) The sub flow path 15s is open to the side face 14D which does not include the apex P among the four side faces 14D. With this configuration, the projection 72 has an increased strength against the force applied to the projection 72 during puncture into the skin, that is, the force that causes the projection 72 to be inclined to a side where the apex P is located rather than toward the center of the projection 72.
(3-4) Since the projection 72 has the pyramidal portion 14 sharpened toward the distal tip end, the projection 72 is easily punctured into the skin. Further, since the projection 72 has the columnar portion 13, the diameter of the opening created by the projection 72 is prevented from expanding near the surface of the skin. As a result, the drug intradermally administered is less likely to leak onto the surface of the skin. Further, since the sub flow path 15s is open to the peripheral surface of the pyramidal portion 14, the effect of preventing drug leakage onto the surface of the skin can be improved.

### [Modified Examples]

The third embodiment can be implemented with modifications as described below.

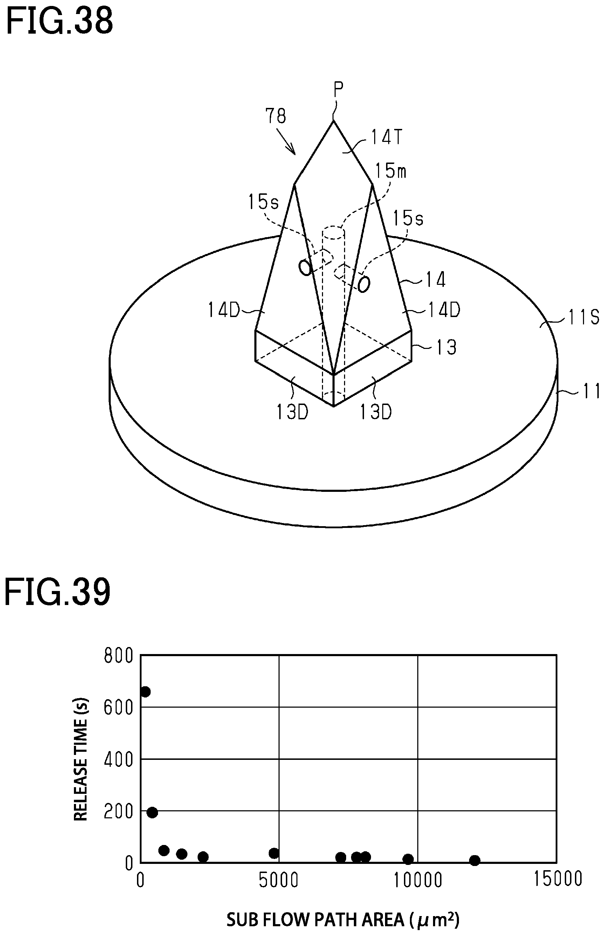

The region defined by each of the main flow path 15m and the sub flow path 15s in a cross-section perpendicular to the extending direction of the flow path is not limited to a circular shape, and may be, for example, a rectangular shape. Further, the shape of the region defined by the main flow path 15m in the above cross-section may be different from the shape of the region defined by the sub flow path 15s in the above cross-section. Regardless of the shape defined by the sub flow path 15s, for smooth release of the drug, the sub flow path area Ss may be 300 µm² or more, and the sub flow path area Ss is preferably 700 µm² or more.
- The shape of the projection 72 is not limited to those described in the above embodiment. For example, as long as the shape of the projection is formed by connecting the bottom of a cone or pyramid to the top of a cylinder or prism and truncating the cone or pyramid obliquely relative to the extending direction thereof, the vertex of the top facet 14T located closest to the base 11 may be located at a position closer to the distal tip end of the projection than the boundary between the cone or pyramid and the cylinder or prism is, or at a position closer to the proximal end of the projection than the boundary is.
   For example, the projection may be formed by truncating a cone or pyramid obliquely relative to the extending direction thereof. Fig. 37 illustrates a projection 77 having a shape formed by truncating a quadrangular prism obliquely relative to the extending direction thereof. In this case, the peripheral surface of the projection 77 is composed of four side faces 17D extending from the support surface 11S in a direction inclined relative to the support surface 11S, and a top facet 17T connected to the four side faces 17D and inclined relative to the support surfaces 11S. Further, when the projection has a shape formed by truncating a cone obliquely relative to the extending direction thereof, the peripheral surface of the projection is composed of a side face, which is a curved surface extending from the support surface 11S, and a top facet connected to the side face and inclined relative to the support surface 11S.
   Further, for example, the projection may have a prismatic shape or a cylindrical shape truncated obliquely relative to the extending direction thereof. Alternatively, the projection may not necessarily have an inclined top facet, and the distal tip end point of the projection may be located at the center of the projection when viewed in a direction facing the support surface 11S. In addition, the top facet of the projection may also be curved, or the peripheral surface of the projection may have a groove or a stepped portion. In short, the only requirement for the projection is that it has a shape able to puncture the skin.
   When the projection has a shape having a dimension in the width direction of the projection gradually increasing from the distal tip end to the proximal end of the projection and thus to the support surface 11S, the strength of the projection near the proximal end is increased. On the other hand, when the projection has the proximal end having a constant dimension in the width direction of the projection, the resistance during puncture of the projection into the skin is reduced compared with the case having a variable dimension in the width direction.
   As long as the projection includes the sub flow path 15s that extends from the main flow path 15m and is open to the peripheral surface of the projection, the position of the opening of the sub flow path 15s and the extending direction of the sub flow path 15s are not limited. For example, the sub flow path 15s may be open to the side face 14D located close to the apex P, or may be open to the top facet 14T or the side face 13D of the columnar portion 13, or at the boundary between the adjacent surfaces that constitute the peripheral surface of the projection. Further, the sub flow path 15s may extend in a direction different from the width direction of the projection, in other words, may extend in the direction inclined relative to the support surface 11S. Alternatively, at the connection between the main flow path 15m and the sub flow path 15s, the distal end of the sub flow path 15s may be located closer to the distal tip end of the projection than to the distal end of the main flow path 15m.
   The projection may include a plurality of sub flow paths 15s that extend from the main flow path 15m and are open to the peripheral surface of the projection. That is, the requirements for the sub flow path area Ss of the third embodiment may also be applied to the administration section of the second embodiment. Fig. 38 illustrates an exemplary projection 78 having two sub flow paths 15s. In order to prevent the strength of the projection from being significantly reduced at a specific position, the heights Hs of the plurality of sub flow paths 15s are preferably different from each other, and the plurality of sub flow paths 15s preferably extend in directions different from each other from the main flow path 15m when viewed in the direction facing the support surface 11S. For example, the side face 14D to which each sub flow path 15s is open is preferably different from the side face 14D to which the other sub flow path 15s is open.
   When the projection includes the plurality of sub flow paths 15s, for smooth release of the drug, the sub flow path area Ss of each of the sub flow paths 15s may be 300 µm² or more, and the sub flow path area Ss is preferably 700 µm² or more.
- Usage of the administration section 70 is not limited to being mounted on the syringe barrel 60. A drug may be supplied into the main flow path 15m of the projection by using a tool other than the syringe barrel 60. Further, the projection may be separated from the base 11 after administration of the drug and left in the skin of the administration target.
- The administration target of liquid drug is not limited to a human, but may also be another animal. Further, the configurations of the above embodiments and the modified examples can be appropriately combined for use.

### [Examples]

The transdermal administration device of the second embodiment will now be described by using specific examples.

### <Production of Transdermal Administration Device>

A polycarbonate was used as a material for the administration section. A plate-shaped base and a structure having a shape formed by connecting a quadrangular pyramid to the top of a quadrangular prism having a square bottom, and truncating the quadrangular pyramid obliquely relative to the extending direction, the structure including a channel therein as a main flow path, were produced by injection molding. Further, a projection was produced by forming an opening as the sub flow path in the structure by laser processing. Transdermal administration devices of Test Examples 1 to 11 having different sub flow path areas Ss were obtained by varying the shape of the opening formed by laser processing. The transdermal administration device includes only the administration section.

For each of the transdermal administration devices of Test Examples 1 to 11, the outline of the projection had the shape shown in Figs. 32A and Fig. 32B, an inclination of the side face of the pyramidal portion relative to the support surface was 80°, the length Lt of the projection was 750 µm, and the length Lc of the columnar portion was 100 µm. Further, for each of the transdermal administration devices of Test Examples 1 to 11, the height Hm of the main flow path was 600 µm, the region defined by the main flow path in a cross-section perpendicular to the extending direction of the main flow path had a circular shape, and the diameter thereof was 100 µm. Further, for each of the transdermal administration devices of Test Examples 1 to 11, the height Hs of the sub flow path was 450 µm, and the sub flow path was open to the side face located farther from the apex P of the projection among the side faces of the pyramidal portion.

In the transdermal administration devices of Test Examples 1 to 6, the region defined by the sub flow path in a cross-section perpendicular to the extending direction of the sub flow path had a circular shape, and the diameter thereof was different among the test examples.

In the transdermal administration devices of Test Examples 7 to 11, the region defined by the sub flow path in a cross-section perpendicular to the extending direction of the sub flow path had a rectangular shape, and a combination of a long side length and a short side length of the rectangular shape was different among the test examples.

### <Evaluation of Liquid Release Capability>

For the transdermal administration devices of Test Examples 1 to 11, liquid release capability was evaluated. Each transdermal administration device was attached to a syringe barrel filled with pure water. The pure water was kept pressed with a 0.2 MPa force so that the pure water was released into the atmosphere from the sub flow path of the projection, and the time required to complete release of 1000 µL pure water was measured. Pressing the pure water was performed by inserting a tube connected to a pressure gauge into an outer cylinder of the syringe barrel as a substitute for a plunger, and supplying a gas into the tube until the pressure inside the tube reached 0.2 MPa.

Table 3 shows the shape of the sub flow path, the sub flow path area Ss, and measurements of the time required to complete release of the above pure water for the transdermal administration devices of Test Examples 1 to 11. Further, the sub flow path area Ss is shown by rounding off the first decimal place.

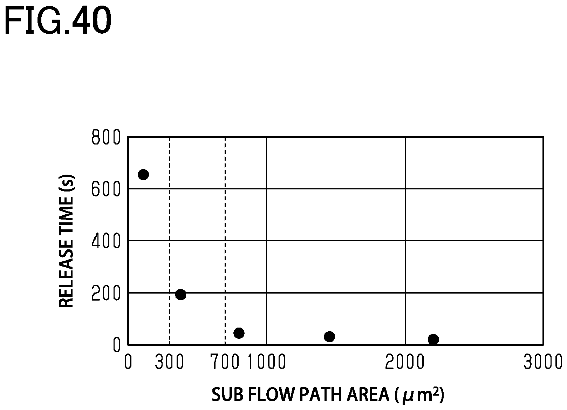

Fig. 39 is a graph representing the results of Table 3, in which the horizontal axis represents the sub flow path area Ss and the vertical axis represents the time required to release pure water. Fig. 40 is an enlarged view of a portion of Fig. 39 indicating the sub flow path area Ss of 3000 µm² or less. That is, Fig. 40 shows the results obtained from Test Examples 1 to 5.

**[Table 3]**

| | Shape of sub flow path | Sub flow path area (µm²) | Release time (s) |
|---|---|---|---|
| Test Example 1 | Circle (12 µm diameter) | 113 | 659 |
| Test Example 2 | Circle (22 µm diameter) | 380 | 197 |
| Test Example 3 | Circle (32 µm diameter) | 804 | 55 |
| Test Example 4 | Circle (43 µm diameter) | 1452 | 39 |
| Test Example 5 | Circle (53 µm diameter) | 2206 | 29 |
| Test Example 6 | Circle (100 µm diameter) | 7854 | 22 |
| Test Example 7 | Rectangle (240 µm long side × 20 µm short side) | 4800 | 41 |
| Test Example 8 | Rectangle (240 µm long side × 40 µm short side) | 9600 | 17 |
| Test Example 9 | Rectangle (240 µm long side × 50 µm short side) | 12000 | 13 |
| Test Example 10 | Rectangle (160 µm long side × 50 µm short side) | 8000 | 21 |
| Test Example 11 | Rectangle (120 µm long side × 60 µm short side) | 7200 | 23 |

As shown in Figs. 39 and 40, in Test Example 1 in which the sub flow path area Ss is of the order of 100 µm², the release time is significantly larger compared with the other test examples. When the sub flow path area Ss is in the range of less than 300 µm², the release time drastically decreases with an increase in the sub flow path area Ss. Further, when the sub flow path area Ss is in the range of 300 µm² or more, the release time is kept small compared to the case where the sub flow path area Ss is less than 300 µm², and the change in release time due to an increase in the sub flow path area Ss is gradual. In particular, when the sub flow path area Ss is in the range of 700 µm² or more, the change in release time due to an increase in the sub flow path area Ss is small. In addition, when the magnitude of force pressing pure water was altered in the range of 0.1 MPa or more and 0.3 MPa or less, the same tendency was observed between the sub flow path area Ss and the release time.

From the above results, it was found that, when the sub flow path area Ss is 300 µm² or more, the release time is significantly smaller than the case where the sub flow path area Ss is less than 300 µm², and the drug can be smoothly ejected from the sub flow path. Furthermore, it was found that, when the sub flow path area Ss is 700 µm² or more, the release time is around the minimum, and the drug can be more smoothly ejected from the sub flow path.

## Claims

1. A transdermal administration device comprising:
an administration section including a base having a base surface, and a projection protruding from the base surface and having a peripheral surface, the projection including a flow path which extends through the projection to the peripheral surface and is open to the peripheral surface; and
a support body including a plurality of support sections configured to abut a target of administration, wherein,
when viewed in a direction facing the base surface, the plurality of support sections surround the base surface with a gap between the adjacent support sections and between the base surface and the support sections, and
the transdermal administration device is configured to displace the projection relative to the support sections from a position in a space surrounded by the plurality of support sections toward outside the space in an extending direction of the projection.

2. The transdermal administration device according to claim 1, wherein,
when viewed in a direction facing the base surface,
a circle having a smallest radius among a plurality of first virtual circles, in which all the support sections are located therein, is an outermost circle,
a circle having a largest radius among a plurality of second virtual circles about a center of the base surface, in which none of the support sections are located therein, is an innermost circle, and
a ratio of a sum of areas of regions of the support sections configured to abut the target to an area of a region between the outermost circle and the innermost circle is in a range of 0.2 or more and 0.8 or less.

3. The transdermal administration device according to claim 1 or 2, wherein positions of ends of the support sections configured to abut the target are aligned with each other in the extending direction of the projection.

4. The transdermal administration device according to claim 1 or 2, wherein
the plurality of support sections include a first support section and a second support section, and
a position of an end of the first support sections configured to abut the target is not aligned with a position of an end of the second support section in the extending direction of the projection.

5. The transdermal administration device according to any one of claims 1 to 4, wherein the plurality of support sections form respective parts of a single pattern when viewed in a direction facing the base surface.

6. A transdermal administration device comprising:
a base having a support surface; and
a projection protruding from the support surface and having a peripheral surface, wherein
the projection includes one main flow path which extends through the projection from a proximal end to a distal tip end of the projection and a plurality of sub flow paths which extend from the main flow path toward the peripheral surface and are open to the peripheral surface, and
an end of the main flow path adjacent to the proximal end of the projection in an extending direction of the main flow path is an open end which penetrates the support surface, and an end close to the distal tip end of the projection is closed.

7. The transdermal administration device according to claim 6, wherein
a length of the sub flow path from a center of gravity of a region defined by the opening to the support surface in a direction perpendicular to the support surface is a height of the sub flow path, and
the height of each sub flow path is different from the height of the other of the plurality of sub flow paths.

8. The transdermal administration device according to claim 6 or 7, wherein each sub flow path extends from the main flow path in a direction different from the directions in which the other sub flow paths extend from the main flow path when viewed in a direction facing the support surface.

9. The transdermal administration device according to any one of claims 6 to 8, wherein
the peripheral surface of the projection includes a top facet inclined relative to the support surface, and a plurality of side faces connected to the top facet, and
the sub flow paths are open to the side faces such that the side face to which each sub flow path is open is different from the side faces to which the other of the sub flow paths are open.

10. The transdermal administration device according to any one of claims 6 to 9, wherein
a resistance that a fluid flowing in the main flow path receives per unit length is a main flow path resistance, and
a resistance that a fluid flowing in the sub flow path per unit length is a sub flow path resistance, and
a sum of the sub flow path resistances for all the sub flow paths is equal to the main flow path resistance.

11. A transdermal administration device comprising:
a base having a support surface; and
a projection protruding from the support surface and having a peripheral surface, wherein
the projection includes a main flow path which extends through the projection from a proximal end to a distal tip end of the projection and a sub flow path which extends from the main flow path toward the peripheral surface and is open to the peripheral surface,
an end of the main flow path adjacent to the proximal end of the projection in an extending direction of the main flow path is an open end which penetrates the support surface and an end close to the distal tip end of the projection is closed, and
an area of a region defined by the sub flow path in a cross-section perpendicular to an extending direction of the sub flow path is 300 µm² or more.

12. The transdermal administration device according to claim 11, wherein an area of a region defined by the sub flow path in a cross-section perpendicular to an extending direction of the sub flow path is 700 µm² or more.

13. The transdermal administration device according to claim 11 or 12, wherein an area of a region defined by the sub flow path in a cross-section perpendicular to the extending direction of the sub flow path is 8000 µm² or less.

14. The transdermal administration device according to any one of claims 11 to 13, wherein
the projection includes an apex,
the peripheral surface of the projection includes a top facet which includes a side including the apex and is inclined relative to the support surface, a first side face that shares the side which includes the apex with the top facet, and a second side face that shares the side which does not include the apex with the top facet, and
the sub flow path is open to the second side face.

15. The transdermal administration device according to any one of claims 6 or 14, wherein
the projection includes a columnar portion having a columnar shape extending from the support surface, and a pyramidal portion extending from a top of the columnar portion and sharpened toward a distal tip end of the projection, and
the sub flow path is open to a peripheral surface of the pyramidal portion.
